# FASCICULE DE BREVET EUROPEEN

(11) **EP 0 656 202 B1**
(45) Date de publication et mention de la délivrance du brevet: **06.05.1999**
(21) Numéro de dépôt: 94402746.5
(22) Date de dépôt: 30.11.1994
(51) Int. Cl.: A61K 7/13, C07C 323/36

(54) **Composition tinctoriale contenant des p-phénylènediamines soufrées, p-phénylènediamines soufrées et leur procédé de préparation**
Färbemittel, das schwefelhaltige p-Phenylendiamine enthält, schwefelhaltige p-Phenylendiamine und Verfahren zur Herstellung
Dyeing composition containing sulphurous p-phenylene-diamines, sulphurous p-phenylenediamines and method of preparation

(30) Priorité: 01.12.1993 FR 9314398
(43) Date de publication de la demande: 07.06.1995
(73) Titulaire: L'OREAL, 75008 Paris (FR)
(72) Inventeur: Genet, Alain, F-93600 Aulnay-sous-Bois (FR); Lagrange, Alain, F-77700 Coupvray (FR)
(74) Mandataire: Casalonga, Axel

(56) Documents cités:
- EP-A- 0 166 155
- WO-A-93/16990
- WO-A-93/18739
- LU-A- 50 461
- US-A- 4 239 708

## Description

L'invention est relative au domaine de la teinture d'oxydation des fibres kératiniques, et plus particulièrement aux compositions tinctoriales contenant des p-phénylènediamines soufrées, ainsi qu'aux procédés de teinture les mettant en oeuvre.

On sait que les p-phénylènediamines jouent, dans les teintures d'oxydation des fibres kératiniques et en particulier des cheveux, un rôle très important, qu'elles soient utilisées seules ou en association avec des coupleurs tels que des m-phénylènediamines, des m-aminophénols, des m-diphénols ou des phénols.

Le document WO-A-9318739 décrit l'utilisation de certaines métaphénylènediamines soufrées en position 2 en tant que coupleurs.

Pour qu'un colorant d'oxydation, et en particulier une p-phénylène diamine, puisse être sélectionné pour des teintures capillaires, il faut qu'il présente, lors de son application dans une teinture pour cheveux, une bonne innocuité et il faut également que les nuances conférées aux cheveux soient stables dans le temps et, en particulier, résistent à la lumière, aux intempéries, aux lavages et aux différents traitements que peuvent subir les cheveux pendant la durée qui sépare habituellement deux colorations successives des cheveux.

La demanderesse a découvert de façon surprenante qu'une famille particulière de p-phénylènediamines soufrées présente simultanément, d'une part, une meilleure innocuité à l'application en teinture pour des fibres kératiniques et en particulier des cheveux et, d'autre part, une meilleure stabilité des nuances obtenues au cours du temps, en particulier une bonne stabilité aux intempéries, à la lumière, aux lavages et aux différents traitements que peuvent subir les cheveux par rapport aux p-phénylènediamines alcoxylées correspondantes.

La présente invention a donc pour objet la composition tinctoriale contenant au moins une p-phénylènediamine de cette famille.

L'invention a également pour objet un procédé de teinture pour fibres kératiniques et en particulier pour cheveux humains, utilisant ces p-phénylènediamines.

Un autre objet de l'invention est constitué par de p-soufrées appartenant à cette famille, ainsi que leur procédé de préparation.

L'invention a enfin pour objet les p-nitroanilines utilisées dans la synthèse des p-phénylènediamines susmentionnées, ainsi que leur utilisation en coloration directe des cheveux.

D'autres objets de l'invention apparaîtront à la lecture de la description et des exemples qui suivent.

Les compositions tinctoriales pour fibres kératiniques et en particulier pour cheveux humains, conformes à l'invention, contiennent dans un milieu approprié pour la teinture, au moins un composé de formule (I) ou un sel d'acide correspondant dans des quantités efficaces pour la teinture.

Les composés utilisés dans les compositions selon l'invention répondent à la formule (I) : 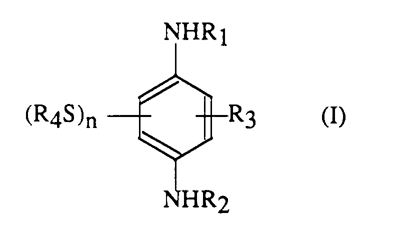 dans laquelle :
R₁ et R₂, identiques ou différents, représentent un atome d'hydrogène, un radical alkyle en C₁-C₄, hydroxyalkyle en C₁-C₄, polyhydroxyalkyle en C₂-C₄, aminoalkyle en C₁-C₄;
R₃ représente un atome d'hydrogène ou d'halogène, un radical alkyle en C₁-C₄;
R₄ représente un radical alkyle en C₁-C₄, hydroxyalkyle en C₁-C₄, polyhydroxyalkyle en C₂-C₄, acétylaminoalkyle en C₁-C₄, aminoalkyle en C₁-C₄, dialkylaminoalkyle en C₁-C₄ éventuellement quaternisé;
n est égal à 1 ou 2; et,
lorsque n=2, les groupements -SR₄ sont en position méta l'un par rapport à l'autre et R₃=H;
et quand n=1 et que R₃ est différent d'hydrogène, alors il est en
position para du groupement -SR₄.

Parmi les composés de formule (I), on peut citer:
- la 2-acétylaminoéthylthio p-phénylènediamine,
- la 2-méthylthio 5-méthyl p-phénylènediamine,
- la 2,6-diméthylthio p-phénylènediamine,
- la 2-éthylthio 1-N-(β-hydroxyéthyl)p-phénylènediamine,
- la 3-éthylthio 1-N-(β-hydroxyéthyl)p-phénylènediamine,
- la 2-β-hydroxyéthylthio p-phénylènediamine,
- la 2-méthylthio p-phénylènediamine,
- la 2-éthylthio p-phénylènediamine,
- la 2-méthylthio 5-chloro p-phénylènediamine,
   ainsi que leurs sels d'addition avec un acide.

Un composé tout particulièrement préféré est le dichlorhydrate de 2-méthylthio p-phénylènediamine.

Les composés de formule (I) sont utilisés de préférence dans les compositions de l'invention à une concentration comprise entre 0,02 et 6% et de préférence comprise entre 0,15 et 5% en poids par rapport au poids total de la composition.

Le pH de la composition tinctoriale selon l'invention est compris entre 8 et 11 et de préférence entre 9 et 11.

Il est ajusté à la valeur désirée à l'aide d'agents alcalinisants bien connus de l'état de la technique, tels que l'ammoniaque, les carbonates alcalins, les alcanolamines tels que les mono-, di- et triéthanolamines, ainsi que leurs dérivés ou les hydroxydes de sodium et de potassium.

Les compositions tinctoriales selon l'invention peuvent contenir un ou plusieurs composés de formule (I). Elles peuvent également contenir d'autres p-phénylènediamines différentes de celles de formule (I), telles que par exemple celles de formule : 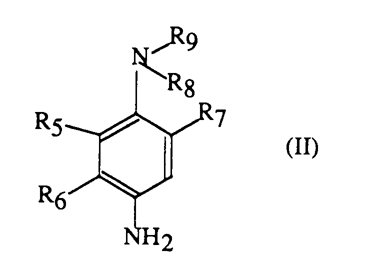 dans laquelle :
R₅, R₆, R₇, identiques ou différents, représentent un atome d'hydrogène ou d'halogène, un radical alkyle, un radical alcoxy, un radical carboxy, sulfo ou hydroxyalkyle en C₁-C₄;
R₈ et R₉, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ou phényle éventuellement substitué en para par un groupement amino; ou bien R₈ et R₉ forment conjointement avec l'atome d'azote auquel ils sont liés, un hétérocycle pipéridino ou morpholino, sous réserve que R₅ ou R₇ représente un atome d'hydrogène lorsque R₈ et R₉ ne représentent pas un atome d'hydrogène, ainsi que les sels de ces composés. Ces radicaux alkyle ou alcoxy ont de préférence 1 à 4 atomes de carbone et désignent notamment les radicaux méthyle, éthyle, propyle, méthoxy et éthoxy.

Parmi les composés de formule (II), on peut plus particulièrement citer la p-phénylènediamine, la p-toluylènediamine, la méthoxyparaphénylènediamine, la chloroparaphénylènediamine, la 2,3-diméthylparaphénylènediamine, la 2,6-diméthylparaphénylènediamine, la 2,6-diéthylparaphénylènediamine, la 2,5-diméthylparaphénylènediamine, la 2-méthyl S-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl, carbamylméthyl)aniline, la 3-méthyl 4-amino N,N-(éthyl, carbamylméthyl)aniline, la 4-amino N,N-(éthyl, β-pipéridinoéthyl)aniline, la 3-méthyl 4-amino N,N-(éthyl, β-pipéridinoéthyl)aniline, la 4-amino N,N-(éthyl, β-morpholinoéthyl)aniline, la 3-méthyl 4-amino N,N-(éthyl,β-morpholinoéthyl)aniline, la 4-amino N,N-(éthyl, β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl, β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-(4-aminophényl)-morpholine, la N-(4-aminophényl)pipéridine, la 2-(β-hydroxyéthyl)paraphénylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylènediamine, la 2-hydroxyméthylparaphénylènediamine, la N,N-diméthyl 3-méthylparaphénylènediamine, la N,N-(éthyl,β-hydroxyéthyl)paraphénylènediamine, la N-(dihydroxypropyl)paraphénylènediamine, la N-(4-aminophényl)paraphénylènediamine, la N-phénylparaphénylènediamine.

Ces p-phénylènediamines peuvent être utilisées, soit sous forme de base libre, soit sous forme de sels, tels que chlorhydrate, bromhydrate ou sulfate.

Les compositions tinctoriales selon l'invention peuvent également contenir des p-aminophénols tels que le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 3-(β-hydroxyéthoxy)4-aminophénol, le 2-aminométhyl 4-aminophénol, le 2-(β-hydroxyéthylaminométhyl)-4-aminophénol, et ceux de formule (III) ci-après : 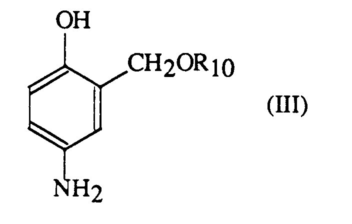 dans laquelle R₁₀ représente un radical alkyle en C₁-C₆, hydroxyalkyle en C₁-C₆, polyhydroxyalkyle en C₂-C₆, halogénoalkyle en C₁-C₆, aminoalkyle en C₂-C₄, aminoalkyle en C₂-C₄ dont l'amine peut être monosubstituée ou disubstituée par un groupement alkyle en C₁-C₄ ou substituée par un groupement dihydroxyalkyle en C₃-C₄, ainsi que leurs sels.

Parmi ces composés de formule (III), on peut en particulier citer :
- le 2-méthoxyméthyl 4-aminophénol,
- le 2-éthoxyméthyl 4-aminophénol,
- le 2-n-propyloxyméthyl 4-aminophénol,
- le 2-isopropyloxyméthyl 4-aminophénol,
- le 2-(β-hydroxyéthoxy)méthyl 4-aminophénol,
- le 2-[(2',2',2'-trifluoroéthoxy)méthyl]4-aminophénol,
   ainsi que leurs sels.

Les compositions tinctoriales selon l'invention peuvent également contenir des bases dites "doubles" qui sont des bis-phénylalkylènediamines, répondant à la formule : 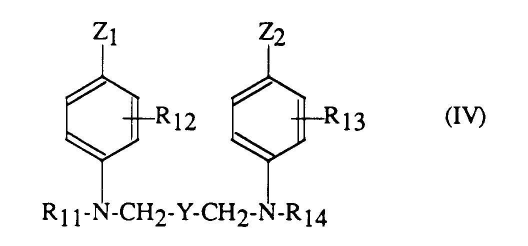 dans laquelle :
Z₁ et Z₂, identiques ou différents, représentent des groupements hydroxyle ou NHR₁₅ où R₁₅ désigne un atome d'hydrogène ou un radical alkyle inférieur;
R₁₂ et R₁₃, identiques ou différents, représentent, soit des atomes d'hydrogène, soit des atomes d'halogène, soit encore des radicaux alkyle;
R₁₁ et R₁₄, identiques ou différents, représentent un atome d'hydrogène, un radical alkyle, hydroxyalkyle ou aminoalkyle, dont le reste amino peut être substitué;
Y représente un radical pris dans le groupe constitué par les radicaux suivants :

   -(CH₂)ₙ-, -(CH₂)ₘ-O-(CH₂)ₘ-,

   -(CH₂)_{q}-CHOH-(CH₂)_{q}-,

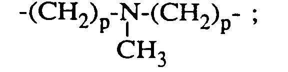

   dans lesquels n est un nombre entier compris entre 0 et 8 et m, q et p sont des nombres entiers compris entre 0 et 4, cette base pouvant se présenter également sous forme de ses sels d'addition avec des acides.

Les radicaux alkyle ou alcoxy ci-dessus indiqués, désignent de préférence un groupement ayant 1 à 4 atomes de carbone et notamment méthyle, éthyle, propyle, méthoxy et éthoxy.

Parmi les composés de formule (IV), on peut citer le N,N'-bis-(β-hydroxyéthyl)N,N'-bis-(4-aminophényl) 1,3-diamino 2-propanol, la N,N'-bis-(β-hydroxyéthyl)N,N'-bis-(4-aminophényl)éthylènediamine, la N,N'-bis-(4-aminophényl)tétraméthylènediamine, la N,N'-bis-(β-hydroxyéthyl)N,N'-bis-(4-aminophényl)tétraméthylènediamine, la N,N'-bis-(4-méthylaminophényl)tétraméthylènediamine, la N,N'-bis-(éthyl)N,N'-bis-(4-amino 3-méthylphényl)éthylènediamine.

Les compositions tinctoriales selon l'invention peuvent également contenir des orthophénylènediamines et des orthoaminophénols comportant éventuellement des substitutions sur le noyau ou sur des fonctions amines.

Parmi les orthoaminophénols, on peut citer plus particulièrement l'orthoaminophénol, le 6-méthyl 1-hydroxy 2-aminobenzène, le 4-méthyl 1-amino 2-hydroxybenzène, le 4-acétylamino 1-amino 2-hydroxybenzène, ainsi que les sels d'acides de ces composés.

Les compositions tinctoriales, objet de la présente invention, contiennent généralement en association avec les précurseurs "para" constitués par les composés de formule (I) et éventuellement avec d'autres précurseurs de colorants d'oxydation de type para et/ou ortho, des coupleurs qui donnent, par couplage oxydatif avec ces précurseurs, des colorants.

Les colorants sont en particulier des indoanilines, des indamines ou des indophénols diversement nuancés qui contribuent à modifier et à enrichir de reflets les colorations "de fond" conférées aux cheveux par les produits de condensation des précurseurs "para" sur eux-mêmes.

Les coupleurs utilisés généralement en association avec les composés de formule (I) dans les compositions tinctoriales, selon l'invention, sont choisis de préférence parmi les métadiphénols, les métaaminophénols, les métaphénylènediamines, les métaacylaminophénols, les métauréidophénols, les métacarbalcoxyaminophénols, l'α-naphtol, les coupleurs hétérocycliques, les coupleurs possédant un groupe méthylène actif tels que les composés β-cétoniques et les pyrazolones.

Parmi les métadiphénols, on peut citer : la résorcine, la 2-méthylrésorcine, la 5-méthylrésorcine, le 2-chlororésorcinol, le monométhyléther de résorcine, le 2,4-dihydroxyphénoxyéthanol, le 2,4-dihydroxyanisole, ainsi que leurs sels. Ces composés associés aux composés de formule (I) conduisent à des nuances gris beige par couplage oxydatif.

Parmi les métaaminophénols, on peut citer le métaaminophénol, le 2-méthyl 5-aminophénol, le 2-méthyl 5-N-(β-hydroxyéthyl)aminophénol, le 2-méthyl 5-N-(β-mésylaminoéthyl)aminophénol, le 2,6-diméthyl 3-aminophénol, le 2-hydroxy 4-N-(β-hydroxyéthyl)aminoanisole, le 2-chloro 6-méthyl 3-aminophénol, le 2-méthyl 3-aminophénol, le 3-diéthylaminophénol, le 1-hydroxy 2,4-dichloro 3-aminobenzène, le 4-chloro 6-méthyl 3-aminophénol, le 6-chloro 3-trifluoroéthylaminophénol, et leurs sels. Ces coupleurs, utilisés avec les composés de formule (I), conduisent par couplage oxydatif à des nuances pourpres.

Parmi les métaphénylènediamines, on peut citer celles qui répondent à la formule : 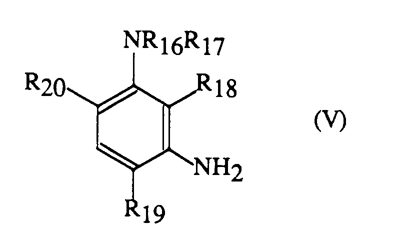 dans laquelle :
R₁₆ et R₁₇ désignent, indépendamment l'un de l'autre, un atome d'hydrogène, un groupement alkyle en C₁-C₄ ou hydroxyalkyle en C₁-C₄;
R₁₈ désigne un atome d'hydrogène ou un groupement alkyle ou alcoxy en C₁-C₄;
R₁₉ désigne un atome d'hydrogène ou un groupement alkyle en C₁-C₄, hydroxyalcoxy en C₁-C₄ ou alcoxy en C₁-C₄;
R₂₀ désigne un atome d'hydrogène ou d'halogène ou un groupement alkyle en C₁-C₄, alcoxy en C₁-C₄, hydroxyalkyle en C₁-C₄, hydroxyalcoxy en C₁-C₄, polyhydroxyalcoxy en C₂-C₄, carboxyalcoxy en C₁-C₄, 2',4'-diaminophénoxyalcoxy en C₁-C₄ ou aminoalcoxy en C₁-C₄;
sous réserve que si R₂₀ désigne carboxyalcoxy ou 2',4'-diaminophénoxyalcoxy, alors R₁₆, R₁₇, R₁₈ et R₁₉ désignent de l'hydrogène, ainsi que leurs sels.

Parmi ces métaphénylènediamines, on peut citer la métaphénylène diamine, le 2,4-diaminophénoxyéthanol, le 2,4-diaminoanisole, le 2,4-diméthoxy 1,3-diaminobenzène, le 1,3,5-triméthoxy 2,4-diaminobenzène, le [2-N-(β-hydroxyéthyl)amino 4-amino]-phénoxyéthanol, le 2-amino 4-N-(β-hydroxyéthyl)aminoanisole, le [4-N-(β-hydroxyéthyl) amino 2-amino]-phénoxyéthanol, le 4,6-bis-(β-hydroxyéthoxy)1,3-diaminobenzène, le (2,4-diamino)phényl-β,γ-dihydroxypropyléther, le (2,4-diamino)phényl-α,β-dihydroxypropyléther, le 1-[2,4-diaminophénoxypropyloxy] 2,4-diaminobenzène, l'acide 2,4-diaminophénoxyacétique, la 2,4-diaminophénoxyéthylamine, la 6-méthoxy 3-hydroxyéthylaminoaniline, le 1-éthoxy 2-bis(β-hydroxyéthyl)amino 4-aminobenzène, le 4-méthyl 6-éthoxy 1,3-diaminobenzène et leurs sels.

Ces coupleurs, utilisés avec les composés de formule (I) conduisent par couplage oxydatif à des couleurs bleues.

On peut aussi mentionner comme coupleurs utilisables dans les compositions de l'invention, le méthylènedioxy-3,4 phénol, le méthylènedioxy-3,4-aniline, le bromo-2 méthylènedioxy 4,5-phénol, le chloro-2 méthylènedioxy-4,5 phénol, l'amino-6 benzomorpholine et l'hydroxy-6 benzomorpholine.

On peut enfin mentionner comme coupleurs particulièrement intéressants, les composés hétérocycliques répondant à la formule : 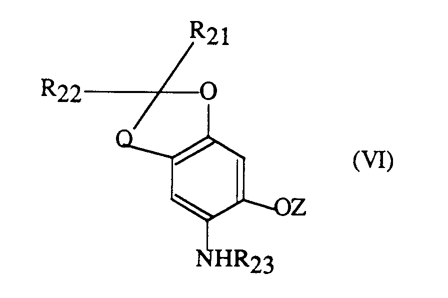 dans laquelle :
R₂₃ représente un atome d'hydrogène, un radical alkyle en C₁-C₄, un radical hydroxyalkyle en C₂-C₄, un radical polyhydroxy alkyle en C₃ à C₆, un radical alcoxyalkyle en C₂-C₆;
Z, indépendamment de R₂₃, représente un radical alkyle en C₁ à C₄, un radical hydroxyalkyle en C₂ à C₄, un radical polyhydroxyalkyle en C₃ à C₆, un radical alcoxyalkyle en C₂-C₆ ou un radical trifluoroéthyle;
R₂₁ et R₂₂ désignent, indépendamment l'un de l'autre, un atome d'hydrogène ou un radical alkyle en C₁ à C₄, ou l'un de ses sels d'addition avec un acide minéral et en particulier l'amino-4 méthoxy-5 méthylènedioxy-1,2 benzène, le (β-hydroxyéthyl)amino-4 méthoxy-5 méthylènedioxy- 1,2 benzène, l'amino-4 (trifluoro-2,2,2-éthoxy)-5 méthylènedioxy-1,2 benzène, le méthylamino-4 méthoxy-5 méthylènedioxy-1,2 benzène, l'amino-4 (β-hydroxyéthoxy)-5 méthylènedioxy-1,2 benzène ou encore l'amino-6 méthoxy-5 méthyl-2 propyl-2 benzodioxol-1,3.

Ces coupleurs, utilisés avec les composés de formule (I) ainsi que l'hydroxy-6 benzomorpholine conduisent, par couplage oxydatif, à des nuances vertes qui sont particulièrement recherchées pour l'obtention de nuances mates pour rabattre les nuances trop rouges et pour apporter une correction éventuelle à la tendance qu'ont certaines teintures à rougir dans le temps.

On peut également utiliser à titre de coupleurs, des composés indoliques tels que le 6-hydroxyindole, le 7-hydroxyindole, le 4-hydroxyindole, le 7-aminoindole, le 5,6-dihydroxyindole, ainsi que leurs dérivés tels que décrits dans les brevets FR 2.636.236, FR 2.654.335, FR 2.654.336, FR 2.659.228, FR 2.664.304, FR 2.664.305 et FR 2.671.722.

Les compositions selon l'invention peuvent également contenir des colorants directs choisis de préférence parmi les colorants azoïques, anthraquinoniques ou les dérivés nitrés de la série benzénique.

L'addition de ces colorants directs aux compositions tinctoriales selon l'invention, permet de nuancer ou d'enrichir en reflets les colorations.

L'ensemble des précurseurs de colorants par oxydation de type para et/ou ortho, ainsi que les coupleurs utilisés dans les compositions tinctoriales conformes à l'invention, représente de préférence de 0,1 à 7% en poids par rapport au poids total de ladite composition.

Les compositions tinctoriales conformes à l'invention contiennent également dans leur forme de réalisation préférée, des agents tensioactifs anioniques, cationiques, non-ioniques, amphotères ou leurs mélanges. Parmi ces agents tensio-actifs, on peut citer les alkylbenzènesulfonates, les alkylnaphtalènesulfonates, les sulfates, les éthersulfates et les sulfonates d'alcools gras, les sels d'ammonium quaternaires, tels que le bromure de triméthylcétylammonium, le bromure de cétylpyridinium, les éthanolamides d'acides gras éventuellement oxyéthylénés, les alcools gras polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0,5 et 55% en poids, et de préférence entre 2 et 50% en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40% en poids, et en particulier entre 2 et 30% en poids par rapport au poids total de la composition.

Les agents épaississants que l'on peut ajouter dans les compositions conformes à l'invention, peuvent être choisis parmi l'alginate de sodium, la gomme arabique, les polymères d'acide acrylique éventuellement réticulés, les dérivés de cellulose, les hétérobiopolysaccharides tels que la gomme de xanthane. On peut également utiliser des agents épaississants minéraux tels que la bentonite.

Ces agents épaississants sont présents de préférence dans des proportions comprises entre 0,1 et 5%, et en particulier entre 0,2 et 3% en poids par rapport au poids total de la composition.

Les agents antioxydants qui peuvent être présents dans les compositions, sont choisis en particulier parmi le sulfite de sodium, l'acide thioglycolique, le bisulfite de sodium, l'acide déhydro-ascorbique, l'hydroquinone, la 2-méthylhydroquinone et l'acide homogentisique. Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des parfums, des tampons, des agents de conditionnement, des agents conservateurs, etc.

Les compositions conformes à l'invention peuvent se présenter sous des formes diverses, telles que sous forme de liquide, de crème, de gel ou sous toute autre forme appropriée pour réaliser une teinture des fibres kératiniques et notamment des cheveux humains. Ces compositions peuvent être conditionnées en flacons aérosols en présence d'un agent propulseur et former des mousses.

Les composés de formule (I) sont utilisés en teinture des fibres kératiniques conformément à l'invention, selon un procédé comprenant l'application sur lesdites fibres du composé de formule (I), en présence ou non d'un ou plusieurs coupleurs et/ou précurseurs de colorant d'oxydation différents de ceux de formule (I) et sont révélés par un agent oxydant.

Les compositions tinctoriales conformes à l'invention, contenant au moins un composé de formule (I) et éventuellement au moins un coupleur et/ou au moins un précurseur de colorant d'oxydation différent de ceux de formule (I), sont utilisées suivant un procédé mettant en oeuvre la révélation par un agent oxydant.

Conformément à ce procédé, on mélange, au moment de l'emploi, la composition tinctoriale décrite ci-dessus avec une solution oxydante en une quantité suffisante pour pouvoir développer une coloration, puis on applique le mélange obtenu sur les fibres kératiniques et en particulier les cheveux humains.

Le pH de la composition appliquée sur les cheveux varie de préférence entre 3 et 11. Il est ajusté à la valeur désirée à l'aide d'agents alcalinisants tels que décrits précédemment ou acidifiants bien connus de l'état de la technique, tels que des acides minéraux ou organiques comme les acides chlorhydrique, tartrique, citrique, phosphorique ou sulfonique.

La solution oxydante peut contenir, à titre d'agent oxydant, l'eau oxygénée, le peroxyde d'urée; des persels, tels que le persulfate d'ammonium; des peracides organiques et leurs sels ou des bromates de métaux alcalins. On utilise de préférence une solution d'eau oxygénée.

Le mélange obtenu est appliqué sur les cheveux et on laisse poser pendant 10 à 40 minutes, de préférence 15 à 30 minutes. Après quoi, on les rince, les lave au shampooing, on les rince à nouveau et on les sèche.

Le composé de formule (I) définie ci-dessus, peut également être mis en oeuvre dans un procédé en plusieurs étapes, consistant dans l'une des étapes, à appliquer le composé de formule (I) et, dans une autre étape, à appliquer une composition tinctoriale contenant au moins un coupleur et/ou au moins un autre précurseur de colorant d'oxydation différent de ceux de formule (I).

L'agent oxydant peut être introduit, juste avant l'application, dans la composition appliquée dans le deuxième temps ou bien être appliqué sur les fibres kératiniques elles-mêmes, dans un troisième temps, les conditions de pose, de pH, de lavage et de séchage étant identiques à celles indiquées ci-dessus.

L'invention a également pour objet de p-phénylènediamines soufrées de la famille de celles répondant à la formule (I).

Ces p-phénylènediamines soufrées conformes à l'invention sont essentiellement caractérisées par le fait qu'elles répondent à la formule : 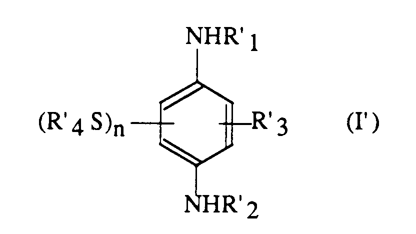 dans laquelle :
R'₁ et R'₂, identiques ou différents, représentent un atome d'hydrogène, un radical alkyle en C₁-C₄, hydroxyalkyle en C₁-C₄, polyhydroxyalkyle en C₂-C₄, aminoalkyle en C₁-C₄;
R'₃ représente un atome d'hydrogène ou d'halogène, un radical alkyle en C₁-C₄;
R'₄ représente un radical alkyle en C₁-C₄, hydroxyalkyle en C₁-C₄, polyhydroxyalkyle en C₂-C₄, acétylaminoalkyle en C₁-C₄, aminoalkyle en C₁-C₄, dialkylaminoalkyle en C₁-C₄ éventuellement quaternisé;
n est égal à 1 ou 2; et,
lorsque n=2, les groupements -SR'₄ sont en position méta l'un par rapport à l'autre et R'₃=H;
et quand n=1 et que R'₃ est différent d'hydrogène, alors il est en position para du groupement -SR'₄;
sous réserve que si R'₁ et R'₂ désignent hydrogène, que n=1 et que R'₄ désigne un radical alkyle en C₁-C₄, alors R'₃ ne peut représenter un atome d'hydrogène ou d'halogène ou le radical propyle ou méthyle; et
sous réserve que quand n=2 et que R'₁, R'₂ et R'₃ désignent un atome d'hydrogène, alors les groupements R'₄ ne peuvent désigner tous les deux en même temps le radical méthyle;
ainsi que les sels d'addition de ces composés avec un acide. Les sels sont choisis en particulier parmi les chlorhydrates, sulfates, tartrates et autres sels cosmétiquement acceptables.

Parmi les composés de formule (I'), on peut citer tout particulièrement :
- la 2-acétylaminoéthylthio p-phénylènediamine,
- la 2,6-diméthylthio p-phénylènediamine,
- la 2-β-hydroxyéthylthio p-phénylènediamine,
- la 2-éthylthio 1-N-(β-hydroxyéthyl)p-phénylènediamine,
- la 3-éthylthio 1-N-(β-hydroxyéthyl)p-phénylènediamine,
   et leurs sels d'addition avec un acide.

Les p-phénylènediamines soufrées répondant à la formule (I') peuvent être préparées selon le procédé comportant essentiellement les étapes suivantes :

1/ l'on traite une halogénonitroaniline de formule (VII) : 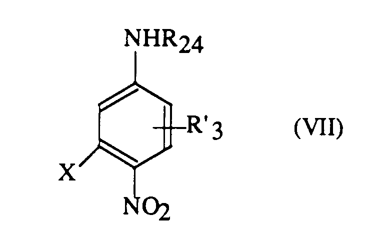 dans laquelle R₂₄ représente un radical acétyle ou un atome d'hydrogène et R'₃ a la signification définie dans la formule (I'); X représente un atome d'halogène, avec un thiol de formule :

ASR'₄ (VIII)

dans laquelle R'₄ a la signification définie dans la formule (I') et A est un atome d'hydrogène ou de sodium pour obtenir la p-nitroaniline de formule (IX) : 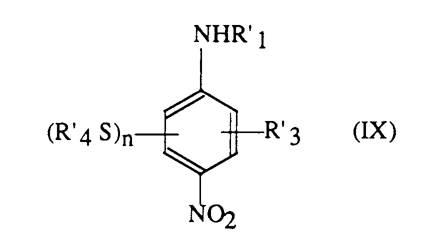 2/ l'on réduit le groupement NO₂ du composé de formule (IX), soit par réduction par l'hydrogène en présence d'un catalyseur, soit par réduction sur le fer dans l'acide acétique ou le zinc en présence d'alcool et de chlorure d'ammonium.

Ce procédé de préparation des composés de formule (I') conforme à l'invention, peut être représenté par les schémas réactionnels suivants.

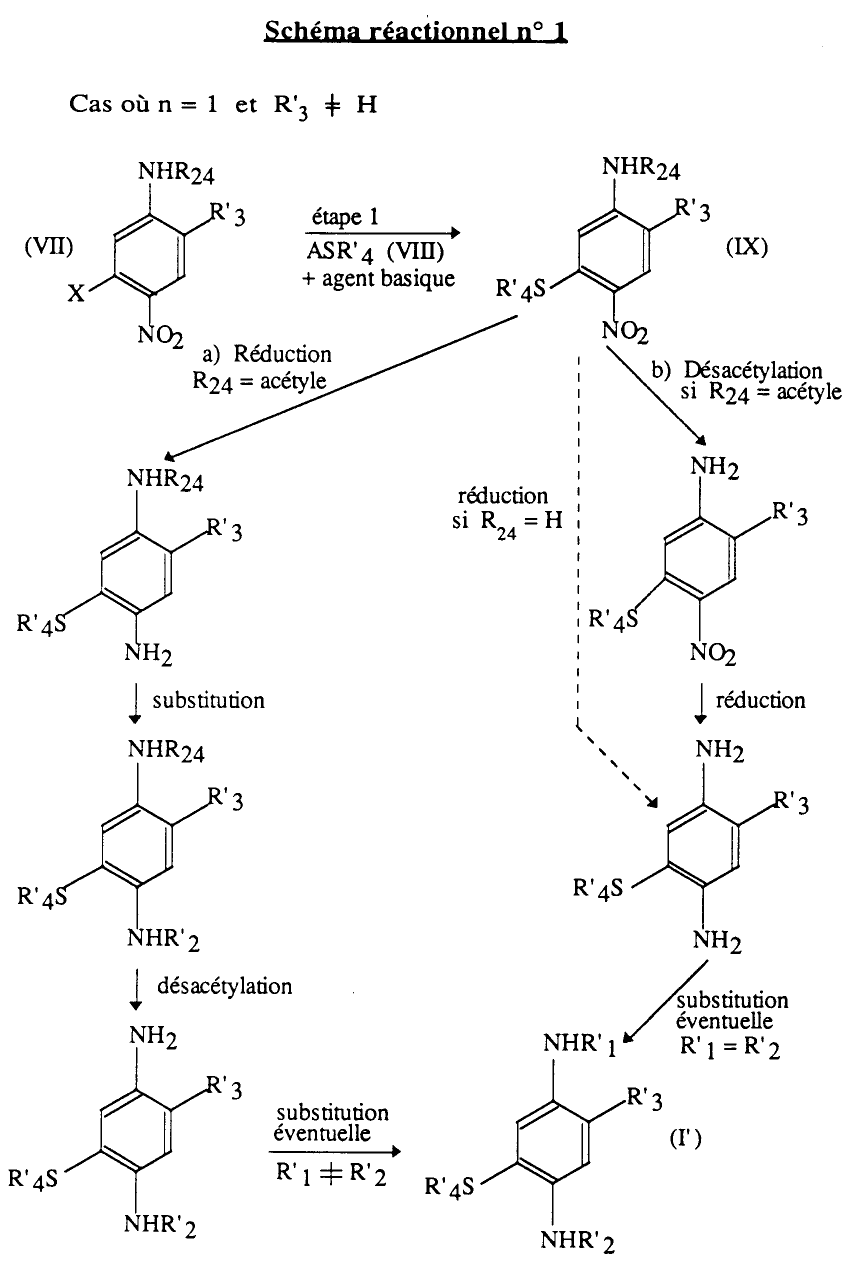

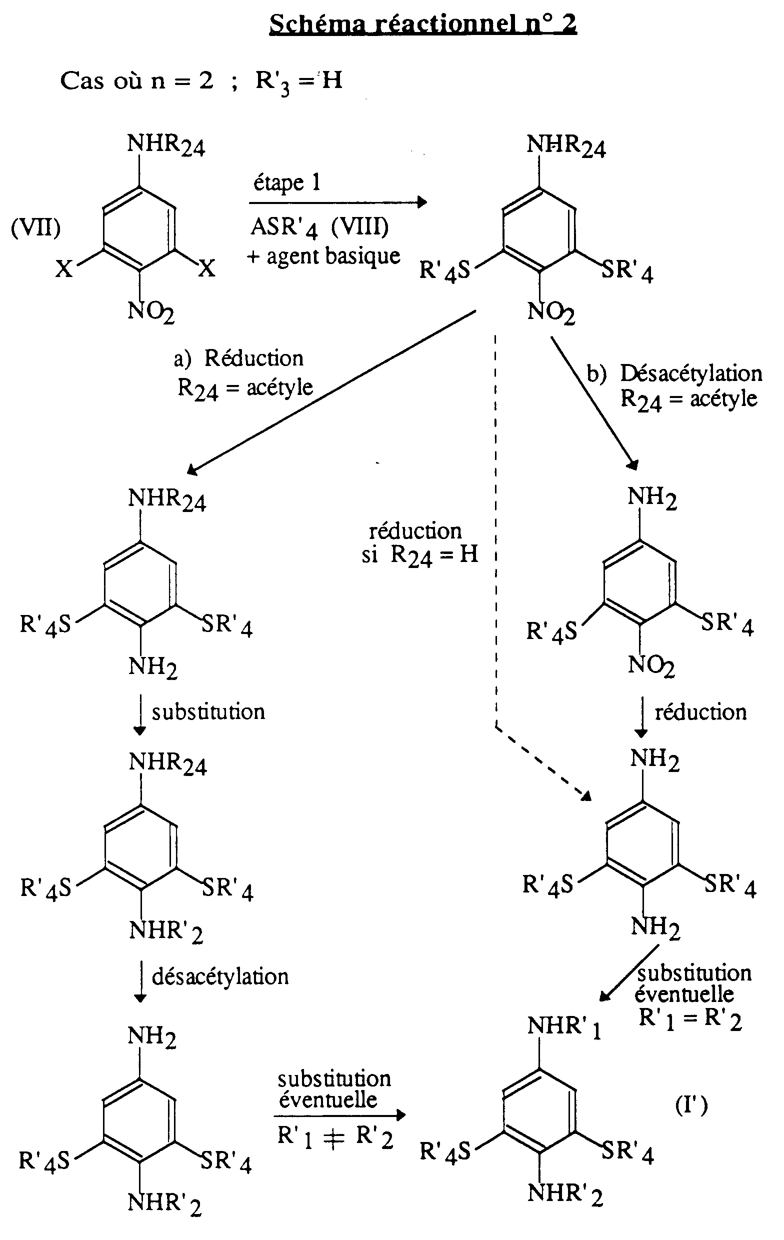

### ETAPE 1

La p-nitroaniline de formule (IX) est préparée par l'action d'un thiol de formule ASR'₄ sur une halogénonitroaniline de formule (VII), dans lequel le radical R'₃ a la signification définie ci-dessus dans la formule (I'). La substitution du ou des groupements halogéno X est effectuée dans un solvant tel que le diméthoxy 1,2-éthane, le diméthylformamide, la N-méthylpyrrolidone, le dioxane, le diméthylsulfoxyde ou l'hexaméthylphosphotriamide. La température de la réaction est comprise entre la température ambiante et la température de reflux du milieu réactionnel. Les "capteurs" de l'acide halohydrique utilisés sont de préférence choisis parmi la soude, la potasse, la triéthylamine, les carbonates de sodium, de potassium et de calcium, ainsi que les sels de sodium des thiols utilisés.

### ETAPES SUIVANTES:

**a)** Si l'on souhaite obtenir un composé de formule (I') dans lequel le radical R'₁ est différent du radical R'₂, on procède alors de la façon suivante :
   - la réduction du groupement NO₂ du composé de formule (IX) dans lequel R₂₄ est acétyle, s'effectue par l'hydrogène en présence d'un catalyseur (Catalytic Hydrogenation Augustine R-L, Marcel DEKKER, Inc. NEW-YORK, 1965) ou par le fer dans l'acide acétique ou le zinc en présence d'alcool et de chlorure d'ammonium;
   - l'amine située en position para du groupement acétylamino est ensuite substituée suivant les méthodes classiques de substitution des amines aromatiques pour obtenir un groupement -NHR'₂, R'₂ ayant les significations définies ci-dessus dans la formule (I');
   - on procède ensuite à la désacétylation du groupement acétylamino selon les méthodes classiques de désacétylation des amines aromatiques pour obtenir un groupement amino que l'on substitue éventuellement comme indiqué ci-dessus, de façon à obtenir un composé de formule (I') dans lequel les radicaux R'₁ et R'₂ sont différents.
**b)** Si l'on souhaite obtenir un composé de formule (I') dans lequel les radicaux R'₁ et R'₂ sont identiques, on procède alors de la façon suivante :
   - on effectue la désacétylation du groupement -NHR₂₄ comme indiqué ci-dessus;
   - on réduit ensuite le groupement NO₂ pour obtenir un composé de formule (I') dans lequel R'₁ = R'₂ = hydrogène;
   - on procède ensuite éventuellement à une substitution des amines aromatiques de façon à obtenir un composé de formule (I') dans lequel R'₁ et R'₂ sont identiques mais différents d'un atome d'hydrogène.

Un autre objet de l'invention est constitué par les composés intermédiaires répondant à la formule : 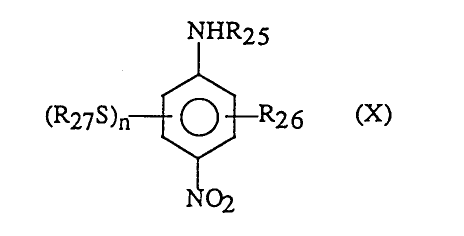 dans laquelle :
R₂₅ représente un atome d'hydrogène, un radical hydroxyalkyle en C₁-C₄ ou acétyle;
R₂₆ représente un atome d'hydrogène, un radical alkyle en C₁-C₄;
R₂₇ représente un radical alkyle en C₁-C₄, un radical acétyl-aminoalkyle en C₁-C₄ ou hydroxyalkyle en C₁-C₄;
quand n=2, les radicaux -SR₂₇ sont en position méta l'un par rapport à l'autre et chacun est en ortho du groupement -NO₂, et R₂₆ désigne l'hydrogène;
quand n=1 et que R₂₆ est différent d'hydrogène, alors le groupement -SR₂₇ est en position para du groupement R₂₆ et en ortho du groupement -NO₂;
sous réserve que lorsque n=1, que le groupement R₂₅ est hydrogène et que R₂₆ désigne hydrogène ou méthyle, alors R₂₇ ne peut désigner méthyle, pour la préparation des p-phénylènediamines soufrées de formule (I').

Parmi ces composés, on peut citer :
- le 2-méthylthio 4-acétylaminonitrobenzène,
- le 2-éthylthio 4-acétylamionitrobenzène,
- le 2-acétylaminoéthylthio 4-acétylaminonitrobenzène,
- le 2-éthylthio 4-aminonitrobenzène,
- le 2,6-diméthylthio 4-aminonitrobenzène,
- le 2-éthylthio 4-N-β-hydroxyéthylaminonitrobenzène,
- le 2-β-hydroxyéthylthio 4-acétylaminonitrobenzène,
- le 2-β-hydroxyéthylthio 4-aminonitrobenzène.

Ces composés peuvent également être utilisés comme colorants pour la teinture directe des cheveux.

Les exemples qui suivent sont destinés à illustrer l'invention.

### EXEMPLES DE PREPARATION

### EXEMPLE 1

### SYNTHESE DU DICHLORHYDRATE DE 2-ETHYLTHIO PARAPHENYLENEDIAMINE

### 1ère étape : Préparation du 2-éthylthio 4-acétylaminonitrobenzène

A une suspension de 25,2 g (0,3 mole) de thioéthylate de sodium dans 220 ml de diméthoxyéthane à température ambiante, on ajoute par portions et sous agitation, en 30 minutes, 46,2 g (0,216 mole) de 2-chloro 4-acétylaminonitrobenzène.

La réaction est exothermique; on maintient la température entre 30 et 35°C. A la fin de l'addition, l'agitation est poursuivie pendant 1 heure, puis on coule le milieu réactionnel (suspension jaune) dans 2 litres d'eau glacée.

Le précipité cristallisé est essoré, réempâté plusieurs fois dans l'eau jusqu'à neutralité, puis séché sous vide sur anhydride phosphorique.

On obtient 48,0 g (0,2 mole) de cristaux jaunes fondant à 148°C (recristallisation de l'acétate d'éthyle bouillant) et dont l'analyse élémentaire calculée pour C₁₀H₁₂N₂O₃ S est :

| % | C | H | N | O | S |
|---|---|---|---|---|---|
| Calculé | 49,99 | 5,03 | 11,66 | 19,98 | 13,34 |
| Trouvé | 49,82 | 5,08 | 11,64 | 20,02 | 13,16 |

### 2ème étape : Préparation du 2-éthylthio 4-aminonitrobenzène

La désacétylation du composé obtenu ci-dessus à la première étape est effectuée en chauffant pendant 10 minutes au bain-marie bouillant 24,0 g (0,1 mole) de ce composé dans un mélange de 50 ml d'acide chlorhydrique concentré à 36% et de 15 ml d'acide acétique. Le milieu réactionnel (suspension) est versé dans 400 ml d'eau glacée et neutralisé par de l'ammoniaque à 20% de NH₃.

Le précipité cristallisé est essoré, lavé à l'eau et recristallisé de l'éthanol à 96°.

On obtient 17,5 g de cristaux jaunes fondant à 97°C et dont l'analyse élémentaire calculée pour C₈H₁₀N₂O₂ S est :

| % | C | H | N | O | S |
|---|---|---|---|---|---|
| Calculé | 48,47 | 5,08 | 14,13 | 16,14 | 16,17 |
| Trouvé | 48,58 | 5,12 | 14,26 | 16,31 | 15,99 |

### 3ème étape : Réduction

A une suspension chauffée au reflux de 1,3 g de chlorure d'ammonium et de 34 g de zinc en poudre fine dans 45 ml d'éthanol à 96° et 10 ml d'eau, on ajoute par portions, de façon à maintenir le reflux sans chauffage, 8,9 g du composé nitré obtenu ci-dessus à la 2ème étape. La réduction est exothermique. Le milieu réactionnel incolore est filtré bouillant. Le filtrat, refroidi dans un bain de glace, est acidifié avec 17 ml d'éthanol absolu chlorhydrique environ 6N.

Le précipité cristallisé est essoré, lavé à l'éther éthylique et séché à 40°C sous vide sur potasse.

On obtient 9,7 g de cristaux blancs de dichlorhydrate de 2-éthylthioparaphénylènediamine fondant avec décomposition à 190-193°C et dont l'analyse élémentaire calculée pour C₈H₁₂N₂ S, 2 HCl est :

| % | C | H | N | S | Cl |
|---|---|---|---|---|---|
| Calculé | 39,84 | 5,85 | 11,61 | 13,29 | 29,40 |
| Trouvé | 39,47 | 5,89 | 11,44 | 12,99 | 29,06 |

### EXEMPLE 2

### SYNTHESE DU DICHLORHYDRATE DE 2-ETHYLTHIO 1-N-(β-HYDROXYETHYL)PARAPHENYLENEDIAMINE

### 1ère étape : Préparation de la 2-éthylthio 4-acétylamino aniline

Selon le mode opératoire décrit à l'exemple 1, 3ème étape, on réduit 22,8 g (0,094 mole) de 2-éthylthio 4-acétylaminonitrobenzène obtenu à l'exemple 1, 1ère étape. Le milieu réactionnel est filtré bouillant. Le filtrat est dilué avec deux volumes d'eau, évaporé partiellement sous pression réduite pour chasser l'éthanol et extrait à l'acétate d'éthyle. La phase acétate d'éthyle est séchée sur sulfate de sodium, filtrée et évaporée à sec.

On obtient un composé huileux (19,0 g) dont l'analyse élémentaire calculée pour C₁₀H₁₄N₂ O S, 1/4 H₂O est :

| % | C | H | N | O | S |
|---|---|---|---|---|---|
| Calculé | 55,92 | 6,80 | 13,04 | 9,31 | 14,93 |
| Trouvé | 55,98 | 6,74 | 13,00 | 9,41 | 14,88 |

### 2ème étape : Préparation du 2-éthylthio 4-acétylamino 1-(β-chlorocarbéthoxy)aminobenzène

A une suspension chauffée au bain-marie bouillant de 18,9 g (0,09 mole) du composé obtenu à l'étape précédente et de 9,1 g de carbonate de calcium dans 95 ml de dioxane, on ajoute goutte à goutte 9,9 ml (0,095 mole) de β-chloroéthylchloroformiate. On maintient le chauffage pendant 1 heure, puis on verse la suspension dans 450 g d'eau glacée. Après acidification avec de l'acide chlorhydrique à 36%, on essore le précipité cristallisé, réempâte dans l'eau et sèche sous vide sur anhydride phosphorique.

On obtient 25,0 g de cristaux blancs fondant à 123°C (recristallisé de l'acétate d'éthyle au reflux) et dont l'analyse élémentaire calculée pour C₁₃H₁₇N₂ O₃ S Cl est :

| % | C | H | N | O | S | Cl |
|---|---|---|---|---|---|---|
| Calculé | 49,29 | 5,41 | 8,84 | 15,15 | 10,12 | 11,19 |
| Trouvé | 49,27 | 5,43 | 8,61 | 15,29 | 9,98 | 11,47 |

### 3ème étape :

L'hydrolyse alcaline est faite en chauffant au bain-marie bouillant pendant 4 heures le mélange de 24,0 g (0,075 mole) du composé obtenu à l'étape précédente, de 70 ml d'alcool à 96°, de 35 ml d'eau et de 70 ml de lessive de soude 10N.

Le mélange réactionnel (huile en suspension) est refroidi dans un bain de glace, dilué avec un volume d'eau, neutralisé à l'acide acétique et évaporé partiellement sous pression réduite pour éliminer l'éthanol.

Après extraction à l'éther éthylique, séchage sur sulfate de sodium, filtration et évaporation à sec, on obtient une huile qui est purifiée par chromatographie sur gel de silice (gradient d'acétate d'éthyle et d'heptane). Le composé purifié huileux est dissous dans l'éthanol absolu.

Après addition de 18 ml d'éthanol absolu chlorhydrique environ 6N, on essore le précipité cristallisé de dichlorhydrate de 2-éthylthio 1-N-(β-hydroxyéthyl)paraphénylènediamine et on sèche sur potasse à 30°C.

On obtient 9,2 g de cristaux blancs fondant avec décomposition à 222-223°C et dont l'analyse élémentaire calculée pour C₁₀H₁₆N₂ O S, 2 HCl est :

| % | C | H | N | O | S | Cl |
|---|---|---|---|---|---|---|
| Calculé | 42,11 | 6,36 | 9,82 | 5,61 | 11,24 | 24,86 |
| Trouvé | 42,08 | 6,38 | 9,85 | 5,64 | 11,49 | 24,63 |

### EXEMPLE 3

### SYNTHESE DU DICHLORHYDRATE DE 3-ETHYLTHIO 1-N-(β-HYDROXYETHYL)PARAPHENYLENEDIAMINE

### 1ére étape : Préparation du 2-éthylthio 4-N-(β-chlorocarbéthoxy) aminonitrobenzène

Ce composé est préparé selon le mode opératoire décrit pour l'exemple 2, étape 2. A partir de 8,4 g (0,0423 mole) de 2-éthylthio 4-aminonitrobenzène (exemple 1, 2ème étape), on obtient 12,8 g de cristaux jaunes fondant à 152°C (recristallisés de l'acétate d'éthyle) et dont l'analyse élémentaire calculée pour C₁₁H₁₃N₂O₄ S Cl est :

| % | C | H | N | O | S | Cl |
|---|---|---|---|---|---|---|
| Calculé | 43,35 | 4,30 | 9,19 | 21,00 | 10,52 | 11,63 |
| Trouvé | 43,48 | 4,46 | 9,01 | 20,87 | 10,54 | 11,58 |

### 2ème étape : Préparation du 2-éthylthio 4-N-(β-hydroxyéthyl) aminonitrobenzène

L'hydrolyse alcaline du composé obtenu à l'étape précédente (11,7 g, 0,0384 mole) est faite en 1/4 d'heure selon le mode opératoire décrit pour l'exemple 2, 3ème étape. Le milieu réactionnel est versé sur 300 g d'eau glacée. Le précipité cristallisé est essoré, réempâté dans l'eau et recristallisé de l'éthanol à 96°.

On obtient 8,2 g de cristaux jaune pâle fondant à 137°C et dont l'analyse élémentaire calculée pour C₁₀H₁₄N₂O₃ S est :

| % | C | H | N | O | S |
|---|---|---|---|---|---|
| Calculé | 49,57 | 5,82 | 11,56 | 19,81 | 13,23 |
| Trouvé | 49,42 | 5,81 | 11,22 | 19,88 | 13,65 |

### 3ème étape :

La réduction du composé obtenu à l'étape précédente (7,8 g, 0,034 mole) est faite selon le mode opératoire décrit dans l'exemple 1, 3ème étape.

On obtient 5,8 g de cristaux blancs du dichlorhydrate fondant avec décomposition à 181-183°C et dont l'analyse élémentaire calculée pour C₁₀H₁₆N₂ O S, 2 HCl est :

| % | C | H | N | O | S | Cl |
|---|---|---|---|---|---|---|
| Calculé | 42,11 | 6,36 | 9,82 | 5,61 | 11,24 | 24,86 |
| Trouvé | 41,98 | 6,39 | 9,77 | 5,50 | 11,26 | 24,80 |

### EXEMPLE 4

### SYNTHESE DU DICHLORHYDRATE DE 2-METHYLTHIO PARAPHENYLENEDIAMINE

Ce composé est préparé selon le mode opératoire décrit pour l'exemple 1, 3ème étape, par réduction de 4,0 g (0,0217 mole) de 2-méthylthio 4-aminonitrobenzène.

On obtient 4,7 g de cristaux blancs du dichlorhydrate attendu fondant avec décomposition à 210-211°C et dont l'analyse élémentaire calculée pour C₇H₁₀N₂ S, 2 HCl est :

| % | C | H | N | S | Cl |
|---|---|---|---|---|---|
| Calculé | 37,01 | 5,32 | 12,33 | 14,12 | 31,21 |
| Trouvé | 36,97 | 5,31 | 12,35 | 14,12 | 31,29 |

### EXEMPLE 5

### SYNTHESE DU DICHLORHYDRATE DE 2-ACETYLAMINOETHYLTHIOPARAPHENYLENEDIAMINE

### 1ère étape : Préparation du 2-acétylaminoéthylthio 4-acétylaminonitrobenzène

On dissout 8,3 g de potasse en poudre dans une solution de 29,8 g (0,25 mole) de N-(2-thioéthyl)acétamide dans 160 ml de diméthoxyéthane chauffée à 40°C. Après refroidissement à 30°C, on ajoute par portions, en 30 minutes, 21,4 g (0,1 mole) de 2-chloro 4-acétyl-aminonitrobenzène en maintenant la température entre 30 et 35°C, puis on chauffe pendant 30 minutes supplémentaires à 70°C. La suspension jaune est versée dans 500 ml d'eau glacée. Le précipité cristallisé est essoré, réempâté dans l'eau et séché à 40°C sur anhydride phosphorique.

On obtient 28,5 g de cristaux jaunes fondant à 195°C (recristallisés de l'éthanol à 96°) et dont l'analyse élémentaire calculée pour C₁₂H₁₅N₃O₄ S est :

| % | C | H | N | O | S |
|---|---|---|---|---|---|
| Calculé | 48,48 | 5,09 | 14,13 | 21,52 | 10,78 |
| Trouvé | 48,56 | 5,18 | 14,06 | 21,65 | 10,74 |

### 2ème étape : Préparation de la 2-acétylaminoéthylthio 4-acétylaminoaniline

La réduction est faite selon le mode opératoire décrit dans l'exemple 2, lère étape, à partir de 28,0 g (0,094 mole) du composé obtenu à l'étape précédente. On obtient des cristaux blancs (17,3 g) fondant à 118°C (recristallisés de l'acétonitrile) et dont l'analyse élémentaire calculée pour C₁₂H₁₇N₃O₂ S est :

| % | C | H | N | O | S |
|---|---|---|---|---|---|
| Calculé | 53,91 | 6,41 | 15,72 | 11,97 | 11,99 |
| Trouvé | 53,48 | 6,47 | 15,60 | 12,56 | 11,85 |

### 3ème étape :

Le composé obtenu à la 2ème étape ci-dessus (16,0 g, 0,06 mole) est agité pendant 6 heures dans 240 ml d'une solution aqueuse normale d'acide chlorhydrique. La solution est refroidie dans un bain de glace, neutralisée à la soude et extraite à l'acétate d'éthyle. La phase acétate d'éthyle est séchée sur sulfate de sodium, filtrée et évaporée à sec sous pression réduite. L'huile obtenue est dissoute dans 100 ml d'éthanol absolu. On acidifie avec 30 ml d'éthanol absolu chlorhydrique environ 6N. Le précipité cristallisé du dichlorhydrate attendu est essoré, lavé à l'éther éthylique et séché sous vide sur potasse.

On obtient 6,2 g de cristaux blancs fondant avec décomposition à 198-200°C et dont l'analyse élémentaire calculée pour C₁₀H₁₅N₃O S, 2 HCl + 1/4 H₂O est :

| % | C | H | N | O | S | Cl |
|---|---|---|---|---|---|---|
| Calculé | 39,67 | 5,83 | 13,88 | 6,61 | 10,59 | 23,42 |
| Trouvé | 40,05 | 5,99 | 13,40 | 7,01 | 9,62 | 23,48 |

### EXEMPLE 6

### SYNTHESE DU DICHLORHYDRATE DE 2-METHYLTHIO 5-CHLOROPARAPHENYLENEDIAMINE

### 1ère étape : Préparation du 2-méthylthio 4-amino 5-chloronitrobenzène

A une suspension de 18,9 g (0,27 mole) de thiométhylate de sodium dans 200 ml de diméthoxyéthane sous agitation à température ambiante, on ajoute par portions 41,4 g (0,2 mole) de 2,5-dichloro 4-aminonitrobenzène en laissant monter la température à 50-55°C et en la maintenant 1/2 heure après la fin de l'addition. On verse le milieu réactionnel sur 2 litres d'eau glacée. Le précipité cristallisé est essoré, réempâté dans l'eau et séché à 40°C sous vide sur anhydride phosphorique.

Après recristallisation de 400 ml d'acétate d'éthyle au reflux, on obtient 29,3 g de cristaux jaunes fondant à 17,4°C et dont l'analyse élémentaire calculée pour C₇H₇N₂O₂ S Cl est :

| % | C | H | N | O | S | Cl |
|---|---|---|---|---|---|---|
| Calculé | 38,45 | 3,23 | 12,81 | 14,63 | 14,66 | 16,21 |
| Trouvé | 38,52 | 3,17 | 12,71 | 14,92 | 14,43 | 16,15 |

### 2ème étape :

La réduction est effectuée selon le mode opératoire décrit pour l'exemple 1, 3ème étape. En partant de 10,9 g (0,05 mole) du composé nitré préparé à l'étape précédente, on obtient 12,4 g du dichlorhydrate du produit attendu fondant avec décomposition à 245-250°C et dont l'analyse élémentaire calculée pour C₇H₉N₂ S Cl, 2 HCl est :

| % | C | H | N | S | Cl |
|---|---|---|---|---|---|
| Calculé | 32,14 | 4,24 | 10,71 | 12,26 | 40,66 |
| Trouvé | 32,24 | 4,29 | 10,81 | 12,19 | 40,68 |

### EXEMPLE 7

### SYNTHESE DU DICHLORHYDRATE DE 2-METHYLTHIO 5-METHYLPARAPHENYLENEDIAMINE

Selon le mode opératoire décrit pour l'exemple 1, 3ème étape, on réduit 9,9 g (0,05 mole) de 2-méthylthio 4-amino 5-méthylnitrobenzène pour obtenir 11,2 g de cristaux blancs fondant avec décomposition à 196-200°C et dont l'analyse élémentaire calculée pour C₈H₁₂N₂ S, 2 HCl est :

| % | C | H | N | S | Cl |
|---|---|---|---|---|---|
| Calculé | 39,84 | 5,85 | 11,61 | 13,29 | 29,40 |
| Trouvé | 39,81 | 5,87 | 11,70 | 13,11 | 29,12 |

### EXEMPLE 8

### SYNTHESE DU DICHLORHYDRATE DE 2,6-DIMETHYLTHIO PARAPHENYLENEDIAMINE

### 1ère étape : Préparation de la 2,6-diméthylthio 4-aminonitrobenzène

Ce composé est préparé selon le mode opératoire décrit pour l'exemple 6, 1ère étape. En partant de 26,9 g (0,13 mole) de 2,6-dichloro 4-aminonitrobenzène et de 0,35 mole de thiométhylate de sodium, on obtient 29,3 g de cristaux jaunes fondant à 198°C et dont l'analyse élémentaire calculée pour C₈H₁₀N₂O₂ S₂ est :

| % | C | H | N | O | S |
|---|---|---|---|---|---|
| Calculé | 41,72 | 4,38 | 12,16 | 13,89 | 27,84 |
| Trouvé | 41,90 | 4,39 | 12,02 | 13,91 | 28,03 |

### 2ème étape :

La réduction est faite selon le mode opératoire décrit à l'exemple 1, 3ème étape. En partant de 10,1 g (0,044 mole) du composé nitré préparé à l'étape précédente, on obtient 11,2 g du dichlorhydrate attendu dont les cristaux blancs fondent avec décomposition à 216-220°C et dont l'analyse élémentaire calculée pour C₈H₁₂N₂S₂, 2 HCl est :

| % | C | H | N | S | Cl |
|---|---|---|---|---|---|
| Calculé | 35,17 | 5,16 | 10,25 | 23,47 | 25,95 |
| Trouvé | 35,06 | 5,24 | 10,13 | 23,25 | 25,90 |

### EXEMPLE 9

### SYNTHESE DU DICHLORHYDRATE DE 2-β-HYDROXYETHYLTHIOPARAPHENYLENEDIAMINE

### 1ère étape : Préparation du 2-β-hydroxyéthylthio 4-acétylaminonitrobenzène

Ce composé est préparé selon le mode opératoire décrit à l'exemple 1, 1ère étape, avec deux modifications : le capteur est le carbonate de potassium et la réaction est effectuée au reflux du diméthoxyéthane.

A partir de 42,9 g (0,2 mole) de 2-chloro 4-acétylaminonitrobenzène et de 23,4 g (0,3 mole) de 2-mercaptoéthanol, on obtient des cristaux jaune pâle (46,6 g) du composé attendu dont le point de fusion est 174°C (recristallisés de l'éthanol à 96°) et dont l'analyse élémentaire calculée pour C₁₀H₁₂N₂O₄S, 1/2 CH₃CH₂OH est :

| % | C | H | N | O | S |
|---|---|---|---|---|---|
| Calculé | 47,30 | 5,41 | 10,03 | 25,78 | 11,48 |
| Trouvé | 46,91 | 5,16 | 10,28 | 25,97 | 11,51 |

### 2ème étape : Préparation du 2-β-hydroxyéthylthio 4-aminonitrobenzène

La désacétylation du composé obtenu ci-dessus à l'étape précédente est effectuée selon le mode opératoire décrit à l'exemple 1, 2ème étape.

A partir de 45,6 g (0,18 mole) du dérivé acétylé, on obtient après recristallisation de l'acétate d'éthyle, des cristaux jaunes (16,1 g) fondant à 146°C et dont l'analyse élémentaire calculée pour C₈H₁₀N₂O₃S est :

| % | C | H | N | O | S |
|---|---|---|---|---|---|
| Calculé | 44,85 | 4,70 | 13,08 | 22,40 | 14,97 |
| Trouvé | 44,91 | 4,78 | 13,12 | 22,55 | 14,82 |

### 3ème étape :

La réduction est faite selon le mode opératoire décrit pour l'exemple 1, 3ème étape.

En partant de 9,8 g (0,0457 mole) du composé nitré obtenu ci-dessus à l'étape précédente, on obtient 9,6 g du dichlorhydrate attendu dont les cristaux blancs fondent avec décomposition à 193-195°C et dont l'analyse élémentaire calculée pour C₈H₁₂N₂OS, 2HCl + 1/4 H₂O est :

| % | C | H | N | O | S | Cl |
|---|---|---|---|---|---|---|
| Calculé | 36,72 | 5,59 | 10,70 | 7,64 | 12,25 | 27,10 |
| Trouvé | 36,77 | 5,54 | 10,59 | 7,86 | 11,99 | 27,33 |

### EXEMPLES DE TEINTURE

### EXEMPLE 1

On prépare la composition tinctoriale suivante :

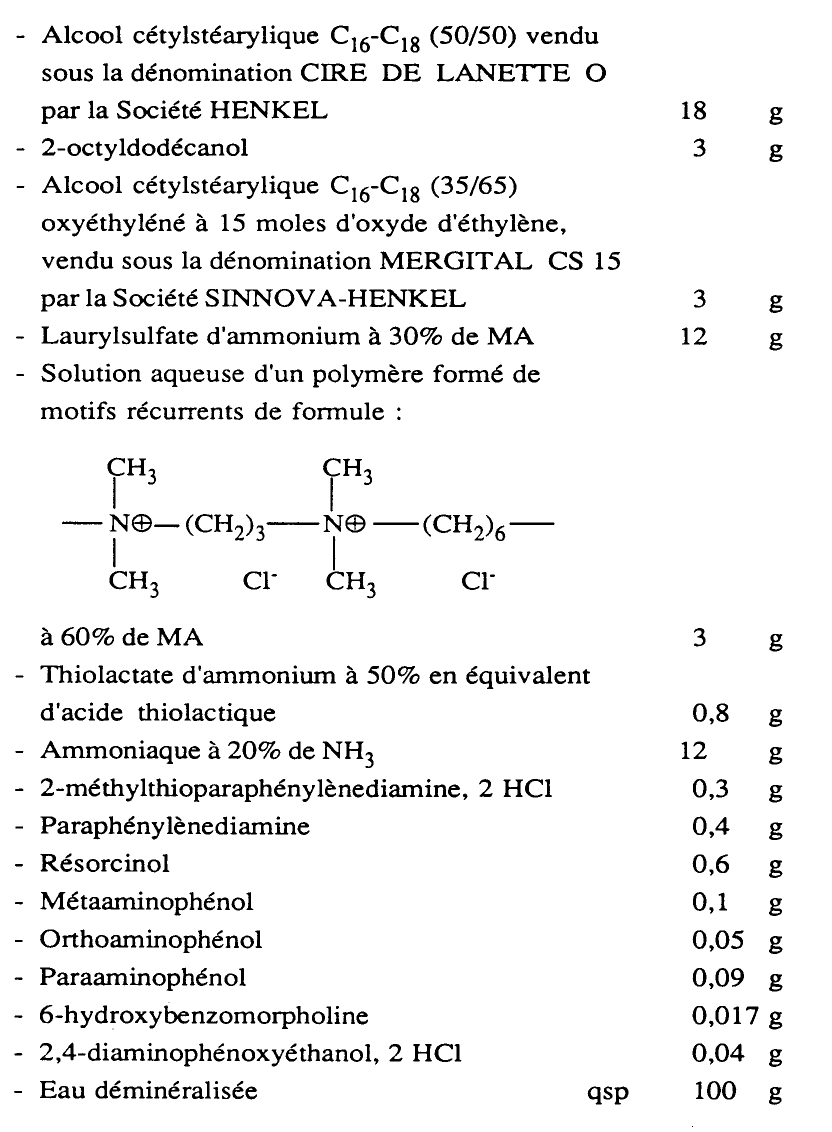

Cette composition est diluée extemporanément avec 1,5 fois son poids d'eau oxygénée à 20 volumes et dont le pH est 3.

Le mélange ainsi réalisé a un pH de 9,8 et est appliqué sur des cheveux naturels à 90% de blancs pendant 30 minutes.

Les cheveux sont ensuite rincés, lavés au shampooing, puis rincés à nouveau, puis séchés.

Les cheveux sont teints dans une nuance châtain clair légèrement doré mat.

### EXEMPLES 2 à 19

On prépare les compositions tinctoriales suivantes :

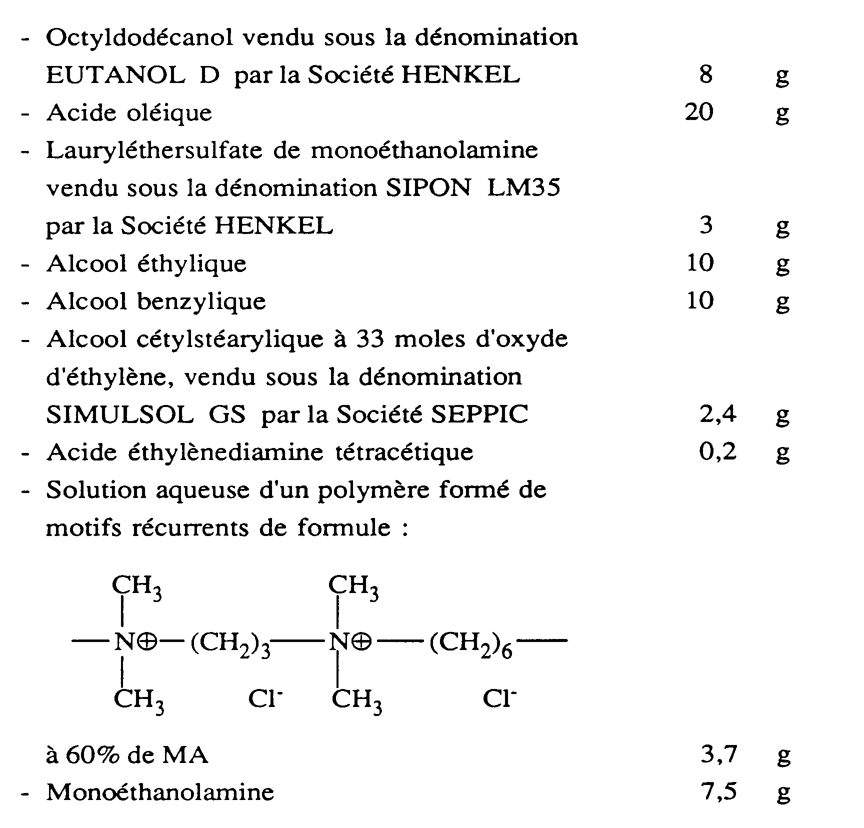

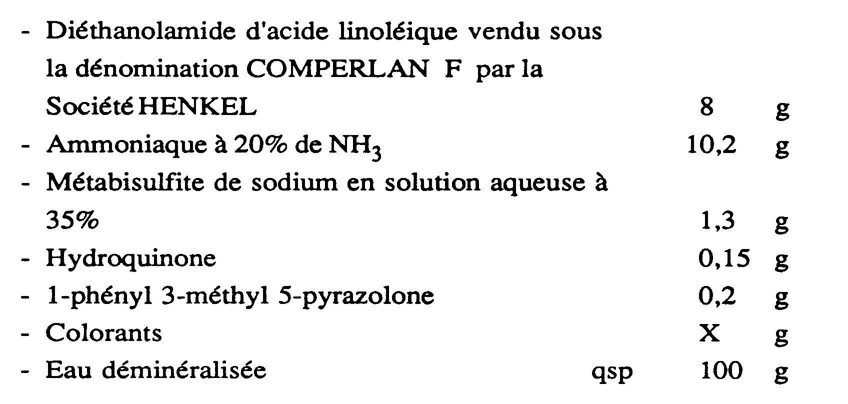

### MODE D'APPLICATION

La composition tinctoriale obtenue est mélangée poids pour poids avec de l'eau oxygénée à 20 volumes et dont le pH est de 3.

Le mélange ainsi réalisé est appliqué sur des cheveux gris à 90% de blancs, permanentés ou non, pendant 30 minutes.

Les cheveux sont ensuite rincés, lavés au shampooing, puis rincés à nouveau et séchés.

Les cheveux sont teints dans les nuances figurant dans les tableaux ci-après :

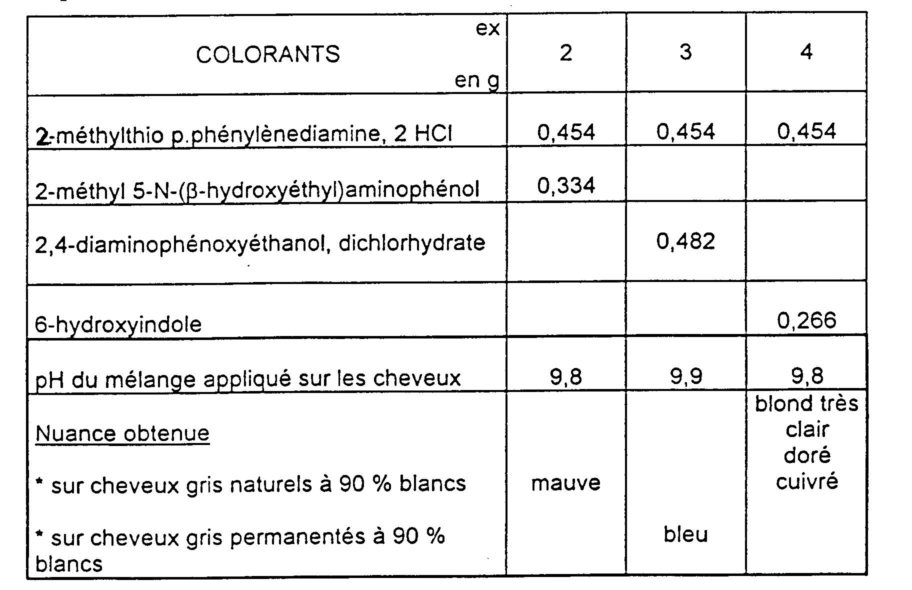

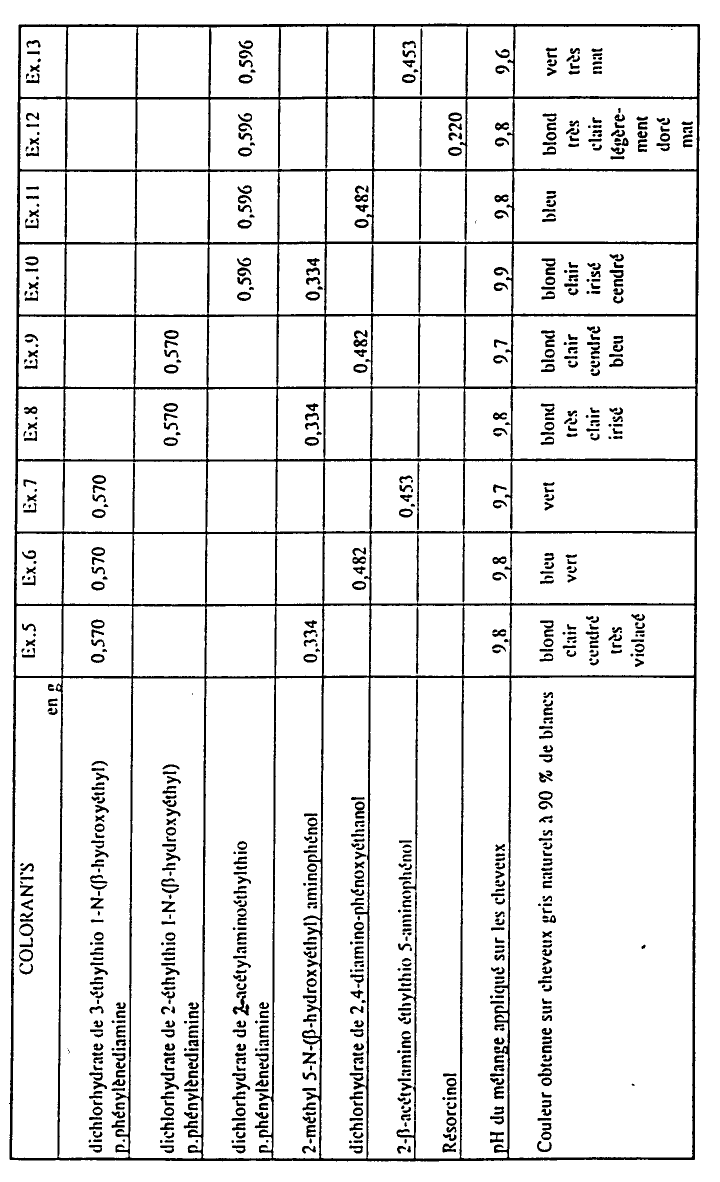

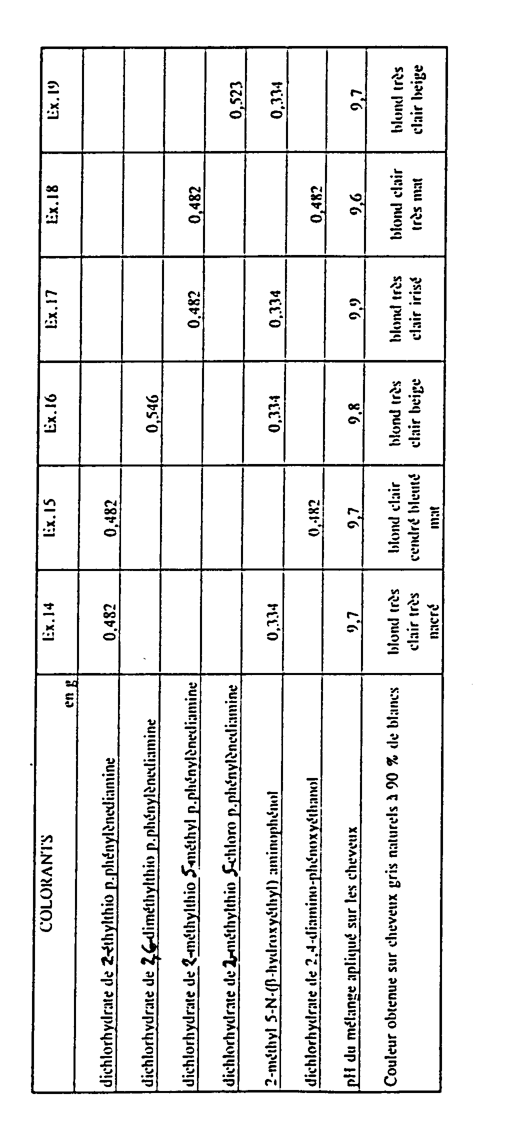

### EXEMPLES 20 à 38

On prépare les compositions tinctoriales suivantes :

| | |
|---|---|
| - Alcool oléique polyglycérolé à 2 moles de glycérol | 4 g |
| - Alcool oléique polyglycérolé à 4 moles de glycérol (78% de MA) | 5,69 g MA |
| - Acide oléique | 3 g |
| - Amine oléique à 2 moles d'oxyde d'éthylène, vendue sous la dénomination ETHOMEEN O12 par la Société AKZO | 7 g |
| - Laurylaminosuccinamate de diéthylaminopropyle, sel de sodium à 55% de MA | 3 g MA |
| - Alcool oléique | 5 g |
| - Diéthanolamide d'acide oléique | 12 g |
| - Propylèneglycol | 3,5 g |
| - Alcool éthylique | 7 g |
| - Dipropylèneglycol | 0,5 g |
| - Monométhyléther de propylèneglycol | 9 g |
| - Métabisulfite de sodium en solution aqueuse à 35% de MA | 1,3 g |
| - Acétate d'ammonium | 0,8 g |
| - Antioxydant, séquestrant qs | |
| - Parfum, conservateur qs | |
| - Monoéthanolamine pH = 9,8 | |
| - Colorants | X g |
| - Eau déminéralisée qsp | 100 g |

### MODE D'APPLICATION

La composition obtenue est mélangée, poids pour poids, avec de l'eau oxygénée à 20 volumes et dont le pH est ajusté entre 1 et 1,5 par 2,5 g d'acide orthophosphorique pour 100 g d'eau oxygénée.

Le mélange est appliqué sur des cheveux gris naturels à 90% de blancs pendant 30 minutes.

Les cheveux sont ensuite rincés, lavés au shampooing, rincés à nouveau, puis séchés.

Les couleurs obtenues figurent dans les tableaux ci-après.

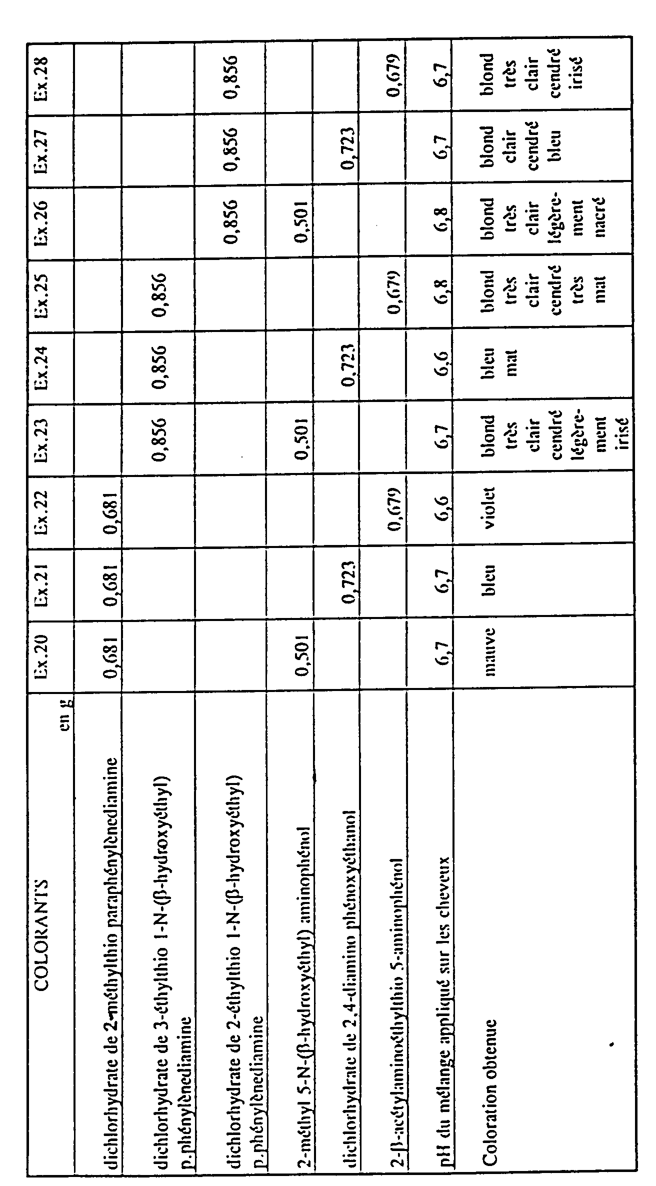

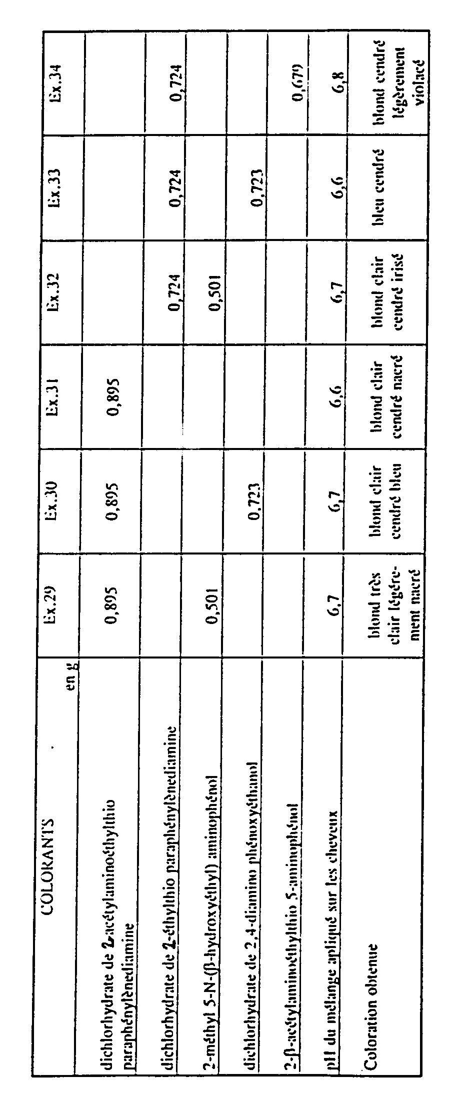

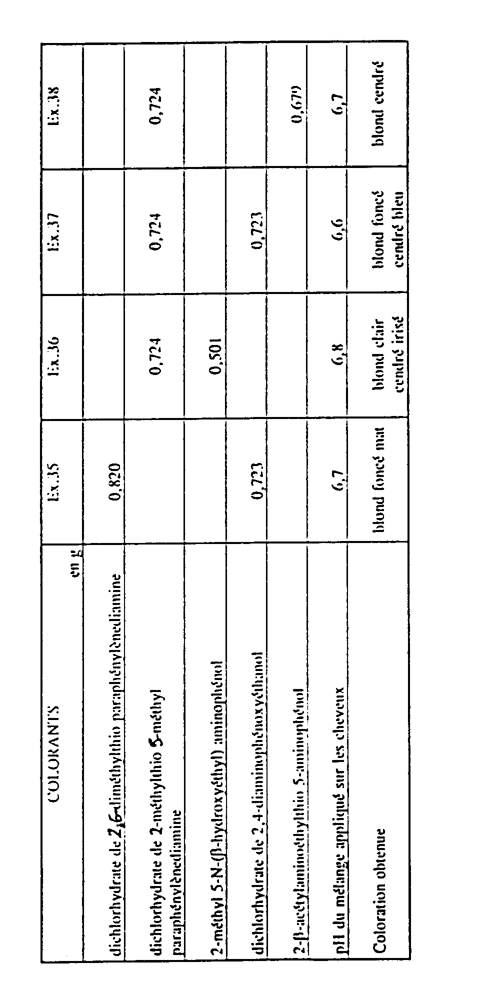

## Revendications

1. Composition tinctoriale pour fibres kératiniques et en particulier pour cheveux humains, caractérisée en ce qu'elle contient dans un milieu approprié pour la teinture, au moins un composé de formule (I) dans des quantités efficaces pour la teinture : 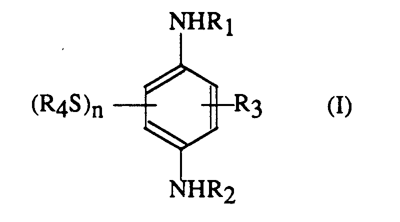 dans laquelle :
R₁ et R₂, identiques ou différents, représentent un atome d'hydrogène, un radical alkyle en C₁-C₄, hydroxyalkyle en C₁-C₄, polyhydroxyalkyle en C₂-C₄, aminoalkyle en C₁-C₄;
R₃ représente un atome d'hydrogène ou d'halogène, un radical alkyle en C₁-C₄;
R₄ représente un radical akyle en C₁-C₄, hydroxyalkyle en C₁-C₄, polyhydroxyalkyle en C₂-C₄, acétylaminoalkyle en C₁-C₄, aminoalkyle en C₁-C₄, dialkylaminoalkyle en C₁-C₄ éventuellement quaternisé;
n est égal à 1 ou 2; et,
lorsque n=2, les groupements -SR₄ sont en position méta l'un par rapport à l'autre et R₃=H;
et quand n=1 et que R₃ est différent d'hydrogène, alors il est en position para du groupement -SR₄;
ainsi que les sels d'addition de ces composés avec un acide.

2. Composition selon la revendication 1, caractérisée en ce que le composé de formule (I) est choisi parmi le groupe constitué de :
- la 2-acétylaminoéthylthio p-phénylènediamine,
- la 2-méthylthio 5-méthyl p-phénylènediamine,
- la 2,6-diméthylthio p-phénylènediamine,
- la 2-éthylthio 1-N-(β-hydroxyéthyl)p-phénylènediamine,
- la 3-éthylthio 1-N-(β-hydroxyéthyl)p-phénylènediamine,
- la 2-β-hydroxyéthylthio p-phénylènediamine,
- la 2-méthylthio p-phénylènediamine,
- la 2-éthylthio p-phénylènediamine,
- la 2-méthylthio 5-chloro p-phénylènediamine,
ainsi que leurs sels d'addition avec un acide.

3. Composition selon la revendication 2, caractérisée en ce que le composé de formule (I) est le dichlorhydrate de 2-méthylthio p-phénylènediamine.

4. Composition selon l'une quelconque des revendications 1 à 3, caractérisée en ce qu'elle contient 0,02 à 6% en poids par rapport au poids total de la composition de composés de formule (I).

5. Composition selon l'une quelconque des revendications 1 à 4, caractérisée en ce qu'elle contient en outre au moins un précurseur de colorant d'oxydation de type para et/ou ortho choisi parmi les p-phénylènediamines différentes de celles de formule (I), les p-aminophénols, les bis-phénylalkylènediamines, les o-phénylènediamines et les o-aminophénols.

6. Composition selon l'une quelconque des revendications 1 à 5, caractérisée en ce qu'elle contient en outre au moins un coupleur choisi parmi les métadiphénols, les métaaminophénols, les métaphénylènediamines, les métaacylaminophénols, les métauréidophénols, les métacarbalcoxyaminophénols, l'α-naphtol, les coupleurs hétérocycliques, les dérivés indoliques, les coupleurs possédant un groupe méthylène actif.

7. Composition selon l'une quelconque des revendications 1 à 6, caractérisée en ce que la composition contient en outre un colorant direct choisi parmi les colorants azoïques, anthraquinoniques ou les dérivés nitrés de la série benzénique.

8. Composition selon l'une quelconque des revendications 1 à 7, caractérisée en ce que l'ensemble des précurseurs de colorants d'oxydation de type para et/ou ortho et des coupleurs est présent dans la composition en une quantité comprise entre 0,1 et 7% en poids par rapport au poids total de ladite composition.

9. Composition selon l'une quelconque des revendications 1 à 8, caractérisée en ce qu'elle contient, en outre, au moins un adjuvant choisi parmi les agents tensio-actifs cationiques, anioniques, non-ioniques, amphotères ou leurs mélanges, en des proportions comprises entre 0,5 et 55% en poids par rapport au poids total de la composition; les solvants organiques en des proportions comprises entre 1 et 40% en poids par rapport au poids total de la composition; les agents épaississants en des proportions comprises entre 0,1 et 5% en poids par rapport au poids total de la composition; les agents antioxydants en des proportions comprises entre 0,01 et 1,5% en poids par rapport au poids total de la composition, les agents de pénétration, les agents séquestrants, les agents de conditionnement, les agents conservateurs, les parfums et les tampons.

10. Composition selon l'une quelconque des revendications 1 à 9, caractérisée en ce qu'elle se présente sous forme de liquide, de crème, de gel ou de toute autre forme appropriée à la teinture, ou peut être conditionnée sous pression en flacon aérosol en présence d'un agent propulseur et former des mousses.

11. Procédé de teinture des fibres kératiniques, en particulier des cheveux humains, caractérisé en ce que l'on applique sur ces fibres une composition tinctoriale contenant dans un milieu approprié pour la teinture, au moins un composé de formule (I) : 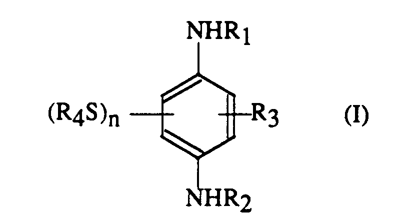 dans laquelle :
R₁ et R₂, identiques ou différents, représentent un atome d'hydrogène, un radical alkyle en C₁-C₄, hydroxyalkyle en C₁-C₄, polyhydroxyalkyle en C₂-C₄, aminoalkyle en C₁-C₄;
R₃ représente un atome d'hydrogène ou d'halogène, un radical alkyle en C₁-C₄;
R₄ représente un radical akyle en C₁-C₄, hydroxyalkyle en C₁-C₄, polyhydroxyalkyle en C₂-C₄, acétylaminoalkyle en C₁-C₄, aminoalkyle en C₁-C₄, dialkylaminoalkyle en C₁-C₄ éventuellement quaternisé;
n est égal à 1 ou 2; et,
lorsque n=2, les groupements -SR₄ sont en position méta l'un par rapport à l'autre et R₃=H;
et quand n=1 et que R₃ est différent d'hydrogène, alors il est en position para du groupement -SR₄;
ainsi que les sels d'addition de ces composés avec un acide;
la couleur étant révélée à l'aide d'un agent oxydant présent dans une solution oxydante en une quantité suffisante pour développer la coloration et mélangée au moment de l'emploi avec la composition contenant le composé de formule (I); le pH de la composition appliquée sur les fibres kératiniques est compris entre 3 et 11.

12. Procédé selon la revendication 11, caractérisé en ce que la composition contenant le composé de formule (I) contient en outre au moins un coupleur.

13. Procédé de teinture des fibres kératiniques, en particulier des cheveux humains, caractérisé en ce que l'on applique sur ces fibres une composition contenant dans un milieu approprié pour la teinture, au moins un composant (I): 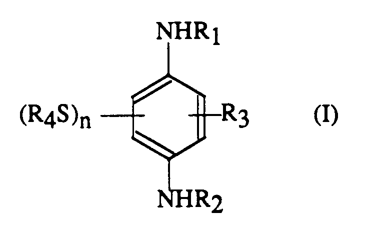 dans laquelle :
R₁ et R₂, identiques ou différents, représentent un atome d'hydrogène, un radical alkyle en C₁-C₄, hydroxyalkyle en C₁-C₄, polyhydroxyalkyle en C₂-C₄, aminoalkyle en C₁-C₄;
R₃ représente un atome d'hydrogène ou d'halogène, un radical alkyle en C₁-C₄;
R₄ représente un radical akyle en C₁-C₄, hydroxyalkyle en C₁-C₄, polyhydroxyalkyle en C₂-C₄, acétylaminoalkyle en C₁-C₄, aminoalkyle en C₁-C₄, dialkylaminoalkyle en C₁-C₄ éventuellement quaternisé;
n est égal à 1 ou 2; et,
lorsque n=2, les groupements -SR₄ sont en position méta l'un par rapport à l'autre et R₃=H;
et quand n=1 et que R₃ est différent d'hydrogène, alors il est en position para du groupement -SR₄;
ainsi que les sels d'addition de ces composés avec un acide;
cette application est suivie ou précédée par l'application sur lesdites fibres d'une composition contenant au moins un coupleur et/ou au moins un précurseur de colorant d'oxydation différent de ceux de formule (I);
la couleur étant développée à l'aide d'un agent oxydant introduit juste avant l'application, dans la composition appliquée dans le deuxième temps, ou bien appliqué directement sur les fibres kératiniques elles-mêmes dans un troisième temps.

14. Utilisation pour la teinture des fibres kératiniques et en particulier des cheveux humains, des composés de formule (I) : 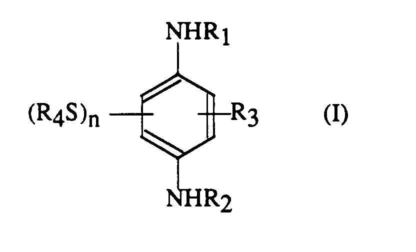 dans laquelle :
R₁ et R₂, identiques ou différents, représentent un atome d'hydrogène, un radical alkyle en C₁-C₄, hydroxyalkyle en C₁-C₄, polyhydroxyalkyle en C₂-C₄, aminoalkyle en C₁-C₄;
R₃ représente un atome d'hydrogène ou d'halogène, un radical alkyle en C₁-C₄;
R₄ représente un radical akyle en C₁-C₄, hydroxyalkyle en C₁-C₄, polyhydroxyalkyle en C₂-C₄, acétylaminoalkyle en C₁-C₄, aminoalkyle en C₁-C₄, dialkylaminoalkyle en C₁-C₄ éventuellement quaternisé;
n est égal à 1 ou 2; et,
lorsque n=2, les groupements -SR₄ sont en position méta l'un par rapport à l'autre et R₃=H;
et quand n=1 et que R₃ est différent d'hydrogène, alors il est en position para du groupement -SR₄;
ainsi que les sels d'addition de ces composés avec un acide.

15. Utilisation selon la revendication 14, caractérisée en ce que le composé de formule (I) est choisi parmi :
- la 2-acétylaminoéthylthio p-phénylènediamine,
- la 2-méthylthio 5-méthyl p-phénylènediamine,
- la 2,6-diméthylthio p-phénylènediamine,
- la 2-éthylthio 1-N-(β-hydroxyéthyl)p-phénylènediamine,
- la 3-éthylthio 1-N-(β-hydroxyéthyl)p-phénylènediamine,
- la 2-β-hydroxyéthylthio p-phénylènediamine,
- la 2-méthylthio p-phénylènediamine,
- la 2-éthylthio p-phénylènediamine,
- la 2-méthylthio 5-chloro p-phénylènediamine,
ainsi que leurs sels d'addition avec un acide.

16. P-phénylènediamines soufrées de formule (I'): 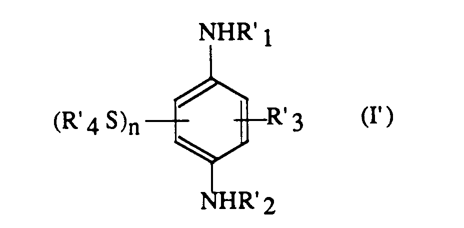 dans laquelle :
R'₁ et R'₂, identiques ou différents, représentent un atome d'hydrogène, un radical alkyle en C₁-C₄, hydroxyalkyle en C₁-C₄, polyhydroxyalkyle en C₂-C₄, aminoalkyle en C₁-C₄;
R'₃ représente un atome d'hydrogène ou d'halogène, un radical alkyle en C₁-C₄;
R'₄ représente un radical alkyle en C₁-C₄, hydroxyalkyle en C₁-C₄, polyhydroxyalkyle en C₂-C₄, acétylaminoalkyle en C₁-C₄, aminoalkyle en C₁-C₄, dialkylaminoalkyle en C₁-C₄ éventuellement quaternisé;
n est égal à 1 ou 2; et,
lorsque n=2, les groupements -SR'₄ sont en position méta l'un par rapport à l'autre et R'₃=H;
et quand n=1 et que R'₃ est différent d'hydrogène, alors il est en position para du groupement -SR'₄;
sous réserve que si R'₁ et R'₂ désignent hydrogène, que n=1 et que R'₄ désigne un radical alkyle en C₁-C₄, alors R'₃ ne peut représenter un atome d'hydrogène ou d'halogène ou le radical propyle ou méthyle; et
sous réserve que quand n=2 et que R'₁ R'₂ et R'₃ désignent un atome d'hydrogène, alors les groupements R'₄ ne peuvent désigner tous les deux en même temps le radical méthyle;
ainsi que les sels d'addition de ces composés avec un acide.

17. p-phénylènediamines soufrées selon la revendication 16, caractérisée en ce qu'elles sont choisies parmi :
- la 2-acétylaminoéthylthio p-phénylènediamine,
- la 2,6-diméthylthio p-phénylènediamine,
- la 2-β-hydroxyéthylthio p-phénylènediamine,
- la 2-éthylthio 1-N-(β-hydroxyéthyl)p-phénylènediamine,
- la 3-éthylthio 1-N-(β-hydroxyéthyl)p-phénylènediamine,
et leurs sels d'addition avec un acide.

18. Composés de formule générale : 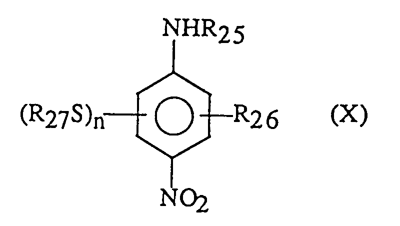 dans laquelle :
R₂₅ représente un atome d'hydrogène, un radical hydroxyalkyle en C₁-C₄ ou acétyle;
R₂₆ représente un atome d'hydrogène, un radical alkyle en C₁-C₄;
R₂₇ représente un radical alkyle en C₁-C₄, un radical acétyl-aminoalkyle en C₁-C₄ ou un radical hydroxyalkyle en C₁-C₄; et,
quand n=2, les radicaux -SR₂₇ sont en position méta l'un par rapport à l'autre et chacun en ortho du groupement -NO₂ et R₂₆ désigne l'hydrogène;
quand n=1 et que R₂₆ est différent d'hydrogène, alors le groupement -SR₂₇ est en position para du groupement R₂₆ et en ortho du groupement -NO₂;
sous réserve que lorsque n=1, que le groupement R₂₅ est hydrogène et que R₂₆ désigne hydrogène ou méthyle, alors R₂₇ ne peut désigner méthyle;
en particulier pour la préparation des p-phénylènediamines soufrées de formule (I') selon la revendication 16.

19. Composé selon la revendication 18, caractérisé en ce qu'il est choisi parmi :
- le 2-méthylthio 4-acétylaminonitrobenzène,
- le 2-éthylthio 4-acétylaminonitrobenzène,
- le 2-acétylaminoéthylthio 4-acétylaminonitrobenzène,
- le 2-éthylthio 4-aminonitrobenzène,
- le 2,6-diméthylthio 4-aminonitrobenzène,
- le 2-éthylthio 4-N-β-hydroxyéthylaminonitrobenzène,
- le 2-β-hydroxyéthylthio 4-acétylaminonitrobenzène,
- le 2-β-hydroxyéthylthio 4-aminonitrobenzène.

## Claims

1. Dye composition for keratin fibres and in particular for human hair, characterized in that it contains, in a medium which is suitable for dyeing, at least one compound of formula (I) in amounts which are effective for dyeing : 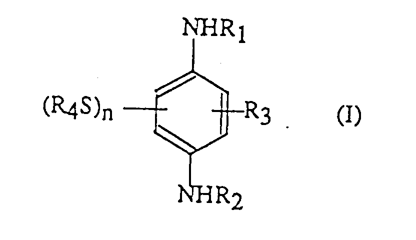 in which :
R₁ and R₂, which may be identical or different, represent a hydrogen atom, a C₁-C₄ alkyl radical, a C₁-C₄ hydroxyalkyl radical, a C₂-C₄ polyhydroxyalkyl radical or a C₁-C₄ aminoalkyl radical;
R₃ represents a hydrogen or halogen atom or a C₁-C₄ alkyl radical;
R₄ represents a C₁-C₄ alkyl radical, a C₁-C₄ hydroxyalkyl radical, a C₂-C₄ polyhydroxyalkyl radical, a C₁-C₄ acetylaminoalkyl radical, a C₁-C₄ aminoalkyl radical or a C₁-C₄ dialkylaminoalkyl radical which is optionally quaternized;
n is equal to 1 or 2; and
when n=2, the groups -SR₄ are in a meta position relative to each other and R₃=H;
and when n=1 and R₃ is other than hydrogen, it is then in the position para to the -SR₄ group;
as well as the addition salts of these compounds with an acid.

2. Composition according to Claim 1, characterized in that the compound of formula (I) is chosen from the group consisting of :
- 2-acetylaminoethylthio-p-phenylenediamine,
- 2-methylthio-5-methyl-p-phenylenediamine,
- 2,6-dimethylthio-p-phenylenediamine,
- 2-ethylthio-1-N-(β-hydroxyethyl)-p-phenylenediamine
- 3-ethylthio-1-N-(β-hydroxyethyl)-p-phenylene-diamine
- 2-(β-hydroxyethylthio)-p-phenylenediamine,
- 2-methylthio-p-phenylenediamine,
- 2-ethylthio-p-phenylenediamine,
- 2-methylthio-5-chloro-p-phenylenediamine,
as well as the addition salts thereof with an acid.

3. Composition according to Claim 2, characterized in that the compound of formula (I) is 2-methylthio-p-phenylenediamine dihydrochloride.

4. Composition according to any one of Claims 1 to 3, characterized in that it contains 0.02 to 6 % by weight, relative to the total weight of the composition, of compounds of formula (I).

5. Composition according to any one of Claims 1 to 4, characterized in that it additionally contains at least one oxidation dye precursor of para and/or ortho type chosen from p-phenylenediamines other than those of formula (I), p-aminophenols, bis-phenylalkylenediamines, o-phenylenediamines and o-aminophenols.

6. Composition according to any one of Claims 1 to 5, characterized in that it additionally contains at least one coupling agent chosen from meta-diphenols, meta-aminophenols, meta-phenylenediamines, meta-acylaminophenols, meta-ureidophenols, meta-carbalkoxyaminophenols, α-naphthol, heterocyclic coupling agents, indole derivatives, and coupling agents possessing an active methylene group.

7. Composition according to any one of Claims 1 to 6, characterized in that the composition additionally contains a direct dye chosen from azo dyes, anthraquinone dyes or nitroderivatives of the benzene series.

8. Composition according to any one of Claims 1 to 7, characterized in that all the oxidation dye precursors of para and/or ortho type and the coupling agents, taken together, are present in the composition in an amount of between 0.1 and 7 % by weight relative to the total weight of the said composition.

9. Composition according to any one of Claims 1 to 8, characterized in that it additionally contains at least one adjuvant chosen from cationic, anionic, nonionic or amphoteric surface-active agents or mixtures thereof in amounts of between 0.5 and 55 % by weight relative to the total weight of the composition; organic solvents in amounts of between 1 and 40 % by weight relative to the total weight of the composition; thickening agents in amounts of between 0.1 and 5 % by weight relative to the total weight of the composition; antioxidants in amounts of between 0.01 and 1.5 % by weight relative to the total weight of the composition, penetration agents, sequestering agents, conditioning agents, preserving agents, fragrances and buffers.

10. Composition according to any one of Claims 1 to 9, characterized in that it is provided in the form of a liquid, a cream, a gel or any other form which is suitable for dyeing, or may be packaged under pressure in an aerosol can in the presence of a propellant and may form foams.

11. Process for dyeing keratin fibres, in particular human hair, characterized in that a dye composition containing, in a medium which is suitable for dyeing, at least one compound of formula (I) : 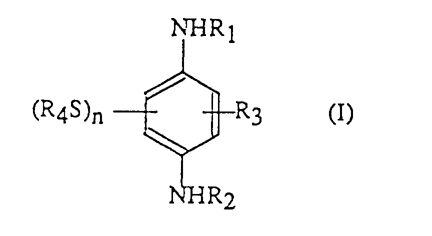 in which :
R₁ and R₂, which may be identical or different, represent a hydrogen atom, a C₁-C₄ alkyl radical, a C₁-C₄ hydroxyalkyl radical, a C₂-C₄ polyhydroxyalkyl radical or a C₁-C₄ aminoalkyl radical;
R₃ represents a hydrogen or halogen atom or a C₁-C₄ alkyl radical;
R₄ represents a C₁-C₄ alkyl radical, a C₁-C₄ hydroxyalkyl radical, a C₂-C₄ polyhydroxyalkyl radical, a C₁-C₄ acetylaminoalkyl radical, a C₁-C₄ aminoalkyl radical or a C₁-C₄ dialkylaminoalkyl radical which is optionally quaternized;
n is equal to 1 or 2; and
when n=2, the groups -SR₄ are in a meta position relative to each other and R₃=H;
and when n=1 and R₃ is other than hydrogen, it is then in the position para to the -SR₄ group;
as well as the addition salts of these compounds with an acid;
is applied to these fibres, the colour being developed using an oxidizing agent which is present in an oxidizing solution in an amount which is sufficient to develop the coloration and is mixed at the time of use with the composition containing the compound of formula (I); the pH of the composition applied to the keratin fibres being between 3 and 11.

12. Process according to Claim 11, characterized in that the composition containing the compound of formula (I) additionally contains at least one coupling agent.

13. Process for dyeing keratin fibres, in particular human hair, characterized in that a composition containing, in a medium which is suitable for dyeing, at least one component (I) : 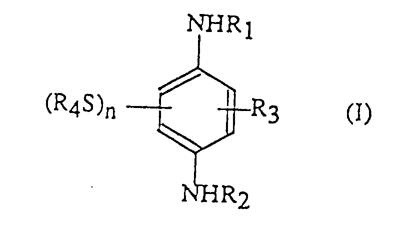 in which :
R₁ and R₂, which may be identical or different, represent a hydrogen atom, a C₁-C₄ alkyl radical, a C₁-C₄ hydroxyalkyl radical, a C₂-C₄ polyhydroxyalkyl radical or a C₁-C₄ aminoalkyl radical;
R₃ represents a hydrogen or halogen atom or a C₁-C₄ alkyl radical;
R₄ represents a C₁-C₄ alkyl radical, a C₁-C₄ hydroxyalkyl radical, a C₂-C₄ polyhydroxyalkyl radical, a C₁-C₄ acetylaminoalkyl radical, a C₁-C₄ aminoalkyl radical or a C₁-C₄ dialkylaminoalkyl radical which is optionally quaternized;
n is equal to 1 or 2; and
when n=2, the groups -SR₄ are in a meta position relative to each other and R₃=H;
and when n=1 and R₃ is other than hydrogen, it is then in the position para to the -SR₄ group;
as well as the addition salts of these compounds with an acid;
is applied to these fibres, this application being followed or preceded by application to the said fibres of a composition containing at least one coupling agent and/or at least one oxidation dye precursor which is different from those of formula (I);
the colour being developed using an oxidizing agent which is introduced just before the application, into the composition which is applied in the second step, or alternatively applied directly to the keratin fibres themselves, in a third step.

14. Use, for dyeing keratin fibres and in particular human hair, of the compounds of formula (I) : 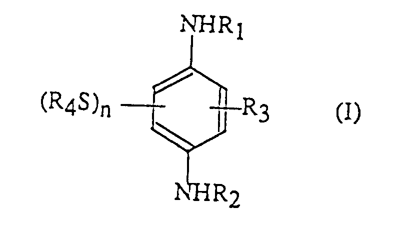 in which :
R₁ and R₂, which may be identical or different, represent a hydrogen atom, a C₁-C₄ alkyl radical, a C₁-C₄ hydroxyalkyl radical, a C₂-C₄ polyhydroxyalkyl radical or a C₁-C₄ aminoalkyl radical;
R₃ represents a hydrogen or halogen atom or a C₁-C₄ alkyl radical;
R₄ represents a C₁-C₄ alkyl radical, a C₁-C₄ hydroxyalkyl radical, a C₂-C₄ polyhydroxyalkyl radical, a C₁-C₄ acetylaminoalkyl radical, a C₁-C₄ aminoalkyl radical or a C₁-C₄ dialkylaminoalkyl radical which is optionally quaternized;
n is equal to 1 or 2; and
when n=2, the groups -SR₄ are in a meta position relative to each other and R₃=H;
and when n-1 and R₃ is other than hydrogen, it is then in the position para to the -SR₄ group;
as well as the addition salts of these compounds with an acid.

15. Use according to Claim 14, characterized in that the compound of formula (I) is chosen from :
- 2-acetylaminoethylthio-p-phenylenediamine,
- 2-methylthio-5-methyl-p-phenylenediamine,
- 2,6-dimethylthio-p-phenylenediamine,
- 2-ethylthio-1-N-(β-hydroxyethyl)-p-phenylene-diamine
- 3-ethylthio-1-N-(β-hydroxyethyl)-p-phenylene-diamine
- 2-(β-hydroxyethylthio)-p-phenylenediamine,
- 2-methylthio-p-phenylenediamine,
- 2-ethylthio-p-phenylenediamine,
- 2-methylthio-5-chloro-p-phenylenediamine,
as well as the addition salts thereof with an acid.

16. sulphur-containing p-phenylenediamines of formula (I') : 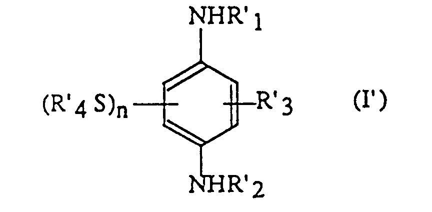 in which
R'₁ and R'₂, which may be identical or different, represent a hydrogen atom, a C₁-C₄, alkyl radical, a C₁-C₄ hydroxyalkyl radical, a C₂-C₄ polyhydroxyalkyl radical or a C₁-C₄ aminoalkyl radical;
R'₃ represents a hydrogen or halogen atom or a C₁-C₄ alkyl radical;
R'₄ represents a C₁-C₄ alkyl radical, a C₁-C₄ hydroxyalkyl radical, a C₂-C₄ polyhydroxyalkyl radical, a C₁-C₄ acetylaminoalkyl radical, a C₁-C₄ aminoalkyl radical or a C₁-C₄ dialkylaminoalkyl radical which is optionally quaternized;
n is equal to 1 or 2; and
when n=2, the groups -SR₄ are in a meta position relative to each other and R'₃=H;
and when n=1 and R'₃ is other than hydrogen, it is then in the position para to the -SR₄ group;
with the proviso that if R'₁ and R'₂ denote hydrogen, n=1 and R'₄ denotes a C₁-C₄ alkyl radical, then R'₃ cannot represent a hydrogen or halogen atom or the propyl or methyl radical; and
with the proviso that when n=2 and R'₁, R'₂ and R'₃ denote a hydrogen atom, then the groups R'₄ cannot both simultaneously denote the methyl radical;
as well as the addition salts of these compounds with an acid.

17. Sulphur-containing p-phenylenediamines according to Claim 16, characterized in that they are chosen from:
- 2-acetylaminoethylthio-p-phenylenediamine,
- 2,6-dimethylthio-p-phenylenediamine,
- 2-(β-hydroxyethylthio)-p-phenylenediamine,
- 2-ethylthio-1-N-(β-hydroxyethyl)-p-phenylene diamine,
- 3-ethylthio-1-N-(β-hydroxyethyl)-p-phenylenediamine,
and the addition salts thereof with an acid.

18. Compounds of general formula : 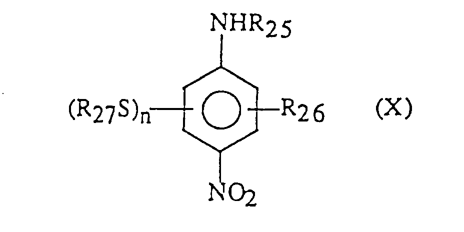 in which :
R₂₅ represents a hydrogen atom, a C₁-C₄ hydroxyalkyl radical or an acetyl radical;
R₂₆ represents a hydrogen atom, a C₁-C₄ alkyl radical;
R₂₇ represents a C₁-C₄ alkyl radical, a C₁-C₄ acetylaminoalkyl radical or-a C₁-C₄ hydroxyalkyl radical; and
when n=2, the radicals -SR₂₇ are in a meta position relative to each other and each is ortho to the -NO₂ group, and R₂₆ denotes hydrogen;
when n=1 and R₂₆ is other than hydrogen, then the group -SR₂₇ is in a position para to the group R₂₆ and ortho to the -NO₂ group;
with the proviso that when n=1, the group R₂₅ is hydrogen and R₂₆ denotes hydrogen or methyl, then R₂₇ cannot denote methyl;
in particular for the preparation of the sulphur-containing p-phenylenediamines of formula (I') according to Claim 16.

19. Compound according to Claim 18, characterized in that it is chosen from :
- 2-methylthio-4-acetylaminonitrobenzene,
- 2-ethylthio-4-acetylaminonitrobenzene,
- 2-acetylaminoethylthio-4-acetylaminonitrobenzene,
- 2-ethylthio-4-aminonitrobenzene,
- 2,6-dimethylthio-4-aminonitrobenzene,
- 2-ethylthio-4-N-(β-hydroxyethylamino)nitrobenzene,
- 2-(β-hydroxyethylthio)-4-acetylaminonitrobenzene,
- 2-(β-hydroxyethylthio)-4-aminonitrobenzene.

## Patentansprüche

1. Färbezusammensetzung für keratinische Fasern und insbesondere für menschliche Haare, dadurch **gekennzeichnet,** daß sie in einem zur Färbung geeigneten Milieu mindestens eine Verbindung der Formel (I) in zur Färbung wirksamen Mengen: 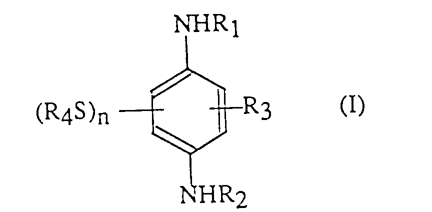 worin gilt:
R₁ und R₂ stellen, gleich oder verschieden, ein Wasserstoffatom, einen C₁₋₄-Alkyl-, C₁₋₄-Hydroxyalkyl-, C₂₋₄-Polyhydroxyalkyl- und einen C₁₋₄-Aminoalkylrest dar;
R₃ stellt ein Wasserstoff- oder Halogenatom oder einen C₁₋₄-Alkylrest dar;
R₄ stellt einen C₁₋₄-Alkyl-, C₁₋₄-Hydroxyalkyl-, C₂₋₄⁻Polyhydroxyalkyl-, C₁₋₄-Acetylaminoalkyl-, C₁₋₄-Aniinoalkyl-oder einen gegebenenfalls quaternierten C₁₋₄-Dialkylaminoalkylrest dar;
n ist 1 oder 2; und
wenn n = 2, liegen die -SR₄-Gruppierungen in meta-Position zueinander vor und R₃ = H;
und wenn n = 1 und sich der Rest R₃ von Wasserstoff unterscheidet, liegt dieser in para-Position zur -SR₄-Gruppierung vor;
sowie die Additionssalze dieser Verbindungen mit einer Säure enthält.

2. Zusammensetzung gemäß Anspruch 1,
dadurch **gekennzeichnet,** daß
die Verbndung der Formel (I) ausgewählt ist aus der Gruppe aus:
2-Acetylaminoethylthio-p-phenylendiamin,
2-Methylthio-5-methyl-p-phenylendiamin,
2,6-Dimethylthio-p-phenylendiamin,
2-Ethylthio-1-N-(β-hydroxyethyl)-p-phenylendiamin,
3-Ethylthio-1-N-(β-hydroxyethyl)-p-phenylendiamin,
2-β-Hydroxyethylthio-p-phenylendiamin,
2-Methylthio-p-phenylendiamin,
2-Ethylthio-p-phenylendiamin,
2-Methylthio-5-chlor-p-phenylendiamin
sowie aus deren Additionssalzen mit einer Säure.

3. Zusammensetzung gemäß Anspruch 2,
dadurch **gekennzeichnet,** daß
die Verbindung der Formel (I) das Dihydrochlorid von 2-Methylthio-p-phenylendiamin ist.

4. Zusammensetzung gemäß einem der Ansprüche 1 bis 3, dadurch **gekennzeichnet,** daß
sie 0,02 bis 6 Gew.% Verbindungen der Formel (I) enthält, bezogen auf das Gesamtgewicht der Zusammensetzung.

5. Zusammensetzung gemäß einem der Ansprüche 1 bis 4, dadurch **gekennzeichnet,** daß
sie mindestens eine Oxidationsfarbstoff-Vorstufenverbindung vom para- und/oder ortho-Typ enthält, ausgewählt aus p-Phenylendiaminen, die sich von denen der Formel (I) unterscheiden, p-Aminophenolen, Bisphenylalkylendiaminen, o-Phenylendiaminen und aus o-Aminophenolen.

6. Zusammensetzung gemäß einem der Ansprüche 1 bis 5, dadurch **gekennzeichnet,** daß
sie ausserdem mindestens einen Kuppler enthält, ausgewählt aus m-Diphenolen, m-Aminophenolen, m-Phenylendiaminen, m-Acylaminophenolen, m-Ureidophenolen, m-Carbalkoxy-aminophenlen, α-Naphthol, heterozyklischen Kupplern, Indolderivaten und aus Kupplern mit einer aktiven Methylengruppe.

7. Zusammensetzung gemäß einem der Ansprüche 1 bis 6, dadurch **gekennzeichnet,** daß
die Zusammensetzung ausserdem einen Direktfarbstoff enthält, ausgewählt aus Azo- und Anthrachinonfarbstoffen oder aus nitrierten Derivaten der Benzolreihe.

8. Zusammensetzung gemäß einem der Ansprüche 1 bis 7, dadurch **gekennzeichnet,** daß
die Gesamtheit der Oxidationsfarbstoff-Vorstufenverbindungen vom para- und/oder ortho-Typ und der Kuppler in der Zusammensetzung in einer Menge von 0,1 bis 7 Gew % vorhanden ist, bezogen auf das Gesamtgewicht der genannten Zusammensetzung.

9. Zusammensetzung gemäß einem der Ansprüche 1 bis 8, dadurch **gekennzeichnet,** daß
sie, ausserdem, mindestens einen Hilfsstoff enthält, ausgewählt aus kationischen, anionischen, nicht-ionischen und amphoteren oberflächenaktiven Mitteln oder deren Mischungen, in Mengenanteilen von 0,5 bis 55 Gew.%, bezogen auf das Gesamtgewicht der Zusammensetzung, aus organischen Lösungsmitteln in Mengenanteilen von 1 bis 40 Gew.%, bezogen auf das Gesamtgewicht der Zusammensetzung, aus Verdickungsmitteln in Mengenanteilen von 0,1 bis 5 Gew.%, bezogen auf das Gesamtgewicht der Zusammensetzung, aus Antioxidantien in Mengenanteilen von 0,01 bis 1,5 Gew.%, bezogen auf das Gesamtgewicht der Zusammensetzung, aus Eindringmitteln, Sequestriermitteln, Konditioniermitteln, Konservierungsmitteln, Parfüm-Produkten und aus Puffermitteln.

10. Zusammensetzung gemäß einem der Ansprüche 1 bis 9, dadurch **gekennzeichnet,** daß
sie in Form einer Flüssigkeit, Creme, eines Gels oder in jeder weiteren zur Färbung geeigneten Form vorliegt oder unter Druck in einem Aerosol-Fläschchen in Gegenwart eines Treibmittels zubereitet vorliegt und Schäume bildet.

11. Verfahren zur Färbung keratinischer Fasern, insbesondere der menschlichen Haare,
dadurch **gekennzeichnet,** daß
man auf diese Fasern eine Färbezusammensetzung aufbringt, die in einem zur Färbung geeigneten Milieu mindestens eine Verbindung der Formel (I): 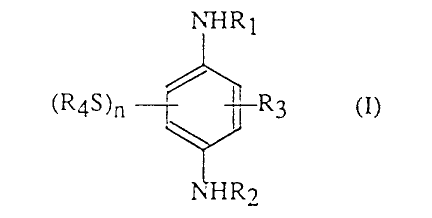 worin gilt:
R₁ und R₂ stellen, gleich oder verschieden, ein Wasserstoffatom, einen C₁₋₄-Alkyl-, C₁₋₄-Hydroxyalkyl-, C₂₋₄-Polyhydroxyalkyl- und einen C₁₋₄-Aminoalkylrest dar;
R₃ stellt ein Wasserstoff- oder Halogenatom oder einen C₁₋₄-Alkylrest dar;
R₄ stellt einen C₁₋₄-Alkyl-, C₁₋₄-Hydroxyalkyl-, C₂₋₄-Polyhydroxyalkyl-, C₁₋₄-Acetylaminoalkyl-, C₁₋₄-Aminoalkyl-oder einen gegebenenfalls quaternierten C₁₋₄-Dialkylaminoalkylrest dar;
n ist 1 oder 2; und
wenn n = 2, liegen die -SR₄-Gruppierungen in meta-Position zueinander vor und R₃ = H;
und wenn n = 1 und sich der Rest R₃ von Wasserstoff unterscheidet, liegt dieser in para-Position zur -SR₄-Gruppierung vor;
sowie die Additionssalze dieser Verbindungen mit einer Säure enthält,
wobei die Farbe mit einem oxidierenden Mittel entwickelt wird, das in einer oxidierenden Lösung in einer zur Entwicklung der Färbung ausreichenden Menge vorhanden ist und zum Zeitpunkt der Anwendung mit der Zusammensetzung vermischt wird, die die Verbindung der Formel (I) enthält, und wobei der pH-Wert der auf die keratinischen Fasern aufgebrachten Zusammensetzung 3 bis 11 beträgt.

12. Verfahren gemäß Anspruch 11,
dadurch **gekennzeichnet,** daß
die Zusammensetzung, die die Verbindung der Formel (I) enthält, ausserdem mindestens einen Kuppler enthält.

13. Verfahren zur Färbung keratinischer Fasern, insbesondere der menschlichen Haare,
dadurch **gekennzeichnet,** daß
man auf diese Fasern eine Zusammensetzung aufbringt, die in einem zur Färbung geeigneten Milieu mindestens einen Bestandteil (I): 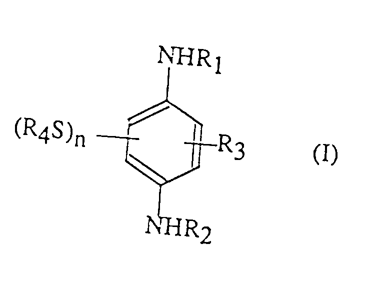 worin gilt:
R₁ und R₂ stellen, gleich oder verschieden, ein Wasserstoffatom, einen C₁₋₄-Alkyl-, C₁₋₄-Hydroxyalkyl-, C₂₋₄-Polyhydroxyalkyl- und einen C₁₋₄-Aminoalkylrest dar;
R₃ stellt ein Wasserstoff- oder Halogenatom oder einen C₁₋₄-Alkylrest dar;
R₄ stellt einen C₁₋₄-Alkyl-, C₁₋₄-Hydroxya1kyl-, C₂₋₄-Polyhydroxyalkyl-, C₁₋₄-Acety1aminoalkyl-, C₁₋₄-Aminoalkyl-oder einen gegebenenfalls quaternierten C₁₋₄-Dialkylaminoalkylrest dar;
n ist 1 oder 2; und
wenn n = 2, liegen die -SR₄-Gruppierungen in meta-Position zueinander vor und R₃ = H;
und wenn n = 1 und sich der Rest R₃ von Wasserstoff unterscheidet, liegt dieser in para-Position zur -SR₄-Gruppierung vor;
sowie die Additionssalze dieser Verbindungen mit einer Säure enthält,
wobei nach oder vor der Aufbringung auf die genannten Fasern eine Zusammensetzung aufgebracht wird, die mindestens einen Kuppler und/oder mindestens eine Oxidationsfarbstoff-Vorstufenverbindung enthält, die sich von denen der Formel (I) unterscheidet,
und wobei die Farbe mit einem oxidierenden Mittel entwickelt wird, das kurz vor der Anwendung in die in der zweiten Stufe aufgebrachte Zusammensetzung eingebracht oder auch direkt auf die keratinischen Fasern selbst in einer dritten Stufe aufgebracht wird.

14. Zur Färbung keratinischer Fasern und insbesondere menschlicher Haare vorgesehene Verwendung der Verbindungen der Formel (I): 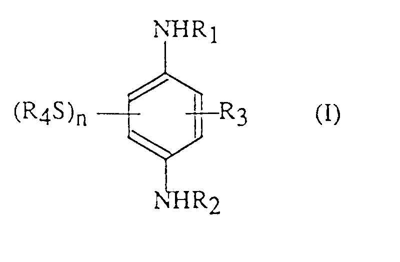 worin gilt:
R₁ und R₂ stellen, gleich oder verschieden, ein Wasserstoffatom, einen C₁₋₄-Alkyl-, C₁₋₄-Hydroxyalkyl-, C₂₋₄-Polyhydroxyalkyl- und einen C₁₋₄-Aminoalkylrest dar;
R₃ stellt ein Wasserstoff- oder Halogenatom oder einen C₁₋₄⁻Alkylrest dar;
R₄ stellt einen C₁₋₄-Alkyl-, C₁₋₄-Hydroxyalkyl-, C₂₋₄-Polyhydroxyalkyl-, C₁₋₄-Acetylaminoalkyl-, C₁₋₄-Aminoalkyl-oder einen gegebenenfalls quaternierten C₁₋₄ -Dialkylaminoalkylrest dar;
n ist 1 oder 2; und
wenn n = 2, liegen die -SR₄-Gruppierungen in meta-Position zueinander vor und R₃ = H;
und wenn n = 1 und sich der Rest R₃ von Wasserstoff unterscheidet, liegt dieser in para-Position zur -SR₄-Gruppierung vor;
sowie der Additionssalze dieser Verbindungen mit einer Säure.

15. Verwendung gemäß Anspruch 14,
dadurch **gekennzeichnet,** daß
die Verbindung der Formel (I) ausgewählt ist aus:
2 -Acetylaminoethylthiop-p-phenylendiamin,
2-Methylthio-5-methyl-p-phenylendiamin,
2,6-Dimethylthio-p-phenylendiamin,
2-Ethylthio-1-N- (β-hydroxyethyl) -p-phenylendiamin,
3-Ethylthio-1-N- (β-hydroxyethyl)-p-phenylendiamin,
2-β-Hydroxyethylthio-p-phenylendiamin,
2-Methylthio-p-phenylendiamin,
2-Ethylthio-p-phenylendiamin,
2-Methylthio-5-chlor-p-phenylendiamin
sowie aus deren Additionssalzen mit einer Säure.

16. Schwefelhaltige p-Phenylendiamine der Formel (I') : 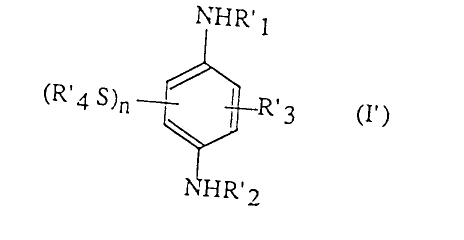 worin gilt:
R'₁ und R'₂ stellen, gleich oder verschieden, ein Wasserstoffatom, eine C₁₋₄-Alkyl-, C₁₋₄-Hydroxyalkyl-, C₂₋₄-Polyhydroxyalkyl- und einen C₁₋₄-Aminoalkylrest dar;
R'₃ stellt ein Wasserstoff- oder Halogenatom oder einen C₁₋₄-Alkylrest dar;
R'₄ stellt einen C₁₋₄-Alkyl-, C₁₋₄-Hydroxyalkyl-, C₂₋₄-Polyhydroxyalkyl-, C₁₋₄-Acetylaminoalkyl-, C₁₋₄-Aminoalkyl-oder einen gegebenenfalls quaternierten C₁₋₄-Dialkylaminoalkylrest dar;
n ist 1 oder 2; und,
wenn n = 2, liegen die -SR'₄-Gruppierungen in meta-Position zueinander vor und R'₃ = H; und wenn
n = 1 und sich der Rest R'₃ von Wasserstoff unterscheidet, liegt dieser dann in para-Position zur -SR₄-Gruppierung vor;
mit der Maßgabe, daß, wenn R'₁ und R'₂ Wasserstoff bedeuten, n = 1 und R'₄ einen C₁₋₄-Alkylrest bedeutet, R'₃ dann nicht
ein Wasserstoff- oder Halogenatom oder den Propyl- oder Methylrest darstellt; und
mit der Maßgabe, daß, wenn n = 2 und R'₁, R'₂ und R'₃ ein Wasserstoffatom bedeuten, die R'₄-Gruppierungen dann nicht beide gleichzeitg den Methylrest bedeuten;
sowie die Additionssalze dieser Verbindungen mit einer Säure.

17. Schwefelhaltige p-Phenylendiamine gemäß Anspruch 16, dadurch **gekennzeichnet,** daß
sie ausgewählt sind aus:
2-Acetylaminoethylthio-p-phenylendiamin,
2,6-Dimethylthio-p-phenylendiamin,
2-β-Hydroxyethylthio-p-phenylendiamin,
2-Ethylthio-1-N-(β-hydroxyethyl)-p-phenylendiamin,
3-Ethylthio-1-N-(β-hydroxyethyl)-p-phenylendiamin
und aus deren Additionssalzen mit einer Säure.

18. Verbindungen der allgemeinen Formel. 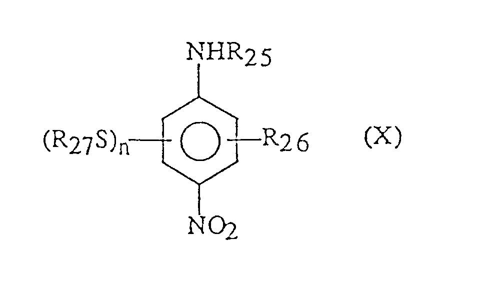 worin gilt:
R₂₅ stellt ein Wasserstoffatom, einen C₁₋₄-Hydroxyalkyl- oder einen Acetylrest dar;
R₂₆ stellt ein Wasserstoffatom oder einen C₁₋₄-Alkylrest dar;
R₂₇ stellt einen C₁₋₄-Alkyl-, C₁₋₄-Acetylaminoalkyl- oder einen C₁₋₄-Hydroxyalkylrest dar;
wenn n = 2, liegen die Reste -SR₂₇ in meta-Position zueinander vor, wobei jeder in ortho-Position zur -NO₂-Gruppe vorliegt, und R₂₆ bedeutet Wasserstoff,
wenn n = 1 und sich R₂₆ von Wasserstoff unterscheidet, liegt die -SR₂₇-Gruppierung in para-Position zur R₂₆-Gruppe und in ortho-Position zur -NO₂-Gruppe vor;
mit der Maßgabe, daß, wenn n=1, die R₂₅-Gruppe Wasserstoff ist und R₂₆ Wasserstoff oder einen Methylrest bedeutet, R₂₇ dann keinen Methylrest bedeutet,
insbesondere zur Herstellung der schwefelhaltigen p-Phenylendiamine der Formel (I') gemäß Anspruch 16.

19. Verbindung gemäß Anspruch 18,
dadurch **gekennzeichnet,** daß
sie ausgewählt ist aus:
2-Methylthio-4-acetylaminonitrobenzol,
2-Ethylthio-4-acetylaminonitrobenzol,
2 -Acetylaminoethylthio-4-acetylaminonitrobenzol,
2 -Ethylthio-4-aminonitrobenzol,
2,6-Dimethylthio-4-aminonitrobenzol,
2-Ethylthio-4-N-β-hydroxyethylaminonitrobenzol,
2-β-Hydroxyethylthio-4-acetylaminonitrobenzol und aus
2-β-Hydroxyethylthio-4-aminonitrobenzol.
